# EUROPEAN PATENT SPECIFICATION

(11) **EP 1 888 746 B1**
(45) Date of publication and mention of the grant of the patent: **17.03.2010**
(21) Application number: 06772459.1
(22) Date of filing: 07.06.2006
(51) Int. Cl.: C12N 15/10, C04B 40/04

(54) **ORDERED MULTI-STEP SYNTHESIS BY NUCLEIC ACID-MEDIATED CHEMISTRY**
GEORDNETE MEHRSCHRITTSYNTHESE MITTELS NUKLEINSÄUREVERMITTELTER CHEMIE
SYNTHÈSE EN PLUSIEURS ÉTAPES ORDONNÉES PAR CHIMIE INDUITE PAR ACIDES NUCLÉIQUES

(30) Priority: 07.06.2005 US 687931 P
(43) Date of publication of application: 20.02.2008
(73) Proprietor: President and Fellows of Harvard College, Cambridge, MA 02138 (US)
(72) Inventor: LIU, David R., Lexington, MA 02420 (US); SNYDER, Thomas M., Palo Alto, CA 94306-1422 (US)
(74) Representative: White, Martin Paul
(86) International application number: PCT/US2006/022172
(87) International publication number: WO 2006/133312

(56) References cited:
- WO-A2-02/103008
- WO-A2-03/078625
- WO-A2-2004/016767
- WO-A2-2004/056994
- WO-A2-2004/110964
- TAMURA KOJI ET AL: "Peptide synthesis with a template-like RNA guide and aminoacyl phosphate adaptors." PROCEEDINGS OF THE NATIONAL ACADEMY OF SCIENCES OF THE UNITED STATES OF AMERICA 22 JUL 2003, vol. 100, no. 15, 22 July 2003 (2003-07-22), pages 8666-8669, XP002420562 ISSN: 0027-8424 cited in the application
- GARTNER Z J ET AL: "TWO ENABLING ARCHITECTURES FOR DNA-TEMPLATED ORGANIC SYNTHESIS" ANGEWANDTE CHEMIE, WILEY-VCH, WEINHEIM, DE, vol. 42, no. 12, 2003, pages 1370-1375, XP001170374 ISSN: 1433-7851
- LI XIAOYU ET AL: "DNA-templated organic synthesis: nature's strategy for controlling chemical reactivity applied to synthetic molecules" ANGEWANDTE CHEMIE. INTERNATIONAL EDITION, VCH VERLAG, WEINHEIM, DE, vol. 43, no. 37, 20 September 2004 (2004-09-20), pages 4848-4870, XP002392408 ISSN: 0570-0833
- GARTNER Z J ET AL: "THE GENERALITY OF DNA-TEMPLATED SYNTHESIS AS A BASIS FOR EVOLVING NON-NATURAL SMALL MOLECULES" JOURNAL OF THE AMERICAN CHEMICAL SOCIETY, AMERICAN CHEMICAL SOCIETY, WASHINGTON, DC, US, vol. 123, 2001, pages 6961-6963, XP001127054 ISSN: 0002-7863 cited in the application
- SNYDER THOMAS M ET AL: "Ordered multistep synthesis in a single solution directed by DNA templates." ANGEWANDTE CHEMIE (INTERNATIONAL ED. IN ENGLISH) 1 DEC 2005, vol. 44, no. 45, 18 November 2005 (2005-11-18), pages 7379-7382, XP002420563 ISSN: 1433-7851

## Description

### FIELD OF THE INVENTION

This invention generally relates to nucleic acid-mediated chemistry. More particularly, this invention relates to ordered multi-step organic synthesis performed by nucleic acid-mediated chemistry.

### BACKGROUND OF THE INVENTION

Many oligomeric natural products including proteins, nonribosomal peptides, and polyketides are biosynthesized in a strictly ordered manner even though all of their constituent building blocks are simultaneously present in the cellular milieu. See, Walsh (2001) SCIENCE 303: 1805-1810. Nature achieves ordered multi-step synthesis by selectively increasing the effective molarity of specific sets of reactants at precise moments during biosynthesis. Compared to the strategy most frequently used by chemists to execute ordered multi-step synthesis - dividing a molecule's construction into a sequence of isolated reactions - nature's single-solution approach is remarkably efficient and elegant while obviating the need for protecting groups.

In the absence of enzymes, ordered multi-step synthesis in a single solution has proven to be a challenge. The ordered oligomerization of monomers on nucleic acid templates has been achieved, but these methods have not allowed the synthesis of non-nucleic-acid structures. See, Kozlov et al. (1999) J. AM. CHEM. SOC. 121: 5856-5859; Kozlov et al. (2000) MOLECULAR BIOLOGY 781-789; . Li et al. (2002) J. AM. CHEM. SOC. 124: 746-747; Rosenbaum et al. (2003) J. AM. CHEM. SOC. 125: 13924-13925; Li et al. (2004) ANGEW. CHEM. INT. ED. 43: 4848-4870.

WO 2003/078625 discloses a method for synthesising templated molecules. The method involves a template, a scaffold functional entity and a functional entity attached to a building block, which, in turn, is attached the template. The scaffold functional entity and the functional entity of the building block are both provided with complementary dimerisation domains that allow the functional entities to come into close proximity when the complementary domains interact with one another. The method may be used for generating libraries of templated molecules which may be selected for biological activity.

WO 2002/103008 discloses a method for synthesising templated molecules, which does not involve ribosome mediated translation of a nucleic acid. The method comprises providing (i) a template comprising a sequence of "n" coding elements, wherein each coding element comprises at least one recognition group capable of recognising a predetermined complementing element, and wherein "n" is an integer of more than 1, and (ii) a plurality of building blocks. Each building block comprises (a) at least one complementing element comprising at least one recognition group capable of recognising a predetermined coding element, (b) at least one functional entity comprising at least one functional group and at least one reactive group, and (c) at least one linker separating the at least one functional entity from the at least one complementing element. Thereafter, each of the coding elements is contacted with a complementing element capable of recognising the coding element under conditions to obtain, optionally, a complementing template, and a templated molecule comprising covalently linked, functional groups by linking, by means of a reaction involving reactive groups, a functional group of at least one functional entity to a functional group of another functional entity. The templated molecule is capable of being linked by means of a linker to the complementing template or to the template that templated the synthesis of the template molecule.

WO 2004/110964 discloses a ribosome-based method for synthesising a templated molecule comprising a plurality of functional groups. The method comprises providing at least one template and a plurality of building blocks. The template comprises a sequence of "n" coding elements selected from the group consisting of first coding elements and second coding elements, wherein each coding element comprises at least one recognition group capable of recognising a predetermined complementing element, and wherein "n" is an integer of at least 3, with the proviso that the template comprises at least 3 first coding elements. The building blocks are selected from the group consisting of first building blocks and second building blocks, with the proviso that at least 3 first building blocks are provided. Each first building block comprises (a) at least one complementing entity comprising a first complementing element comprising at least one recognition group capable of recognising a predetermined first coding element, (b) at least one functional entity comprising at least one functional group and at least one functional entity reactive group, and (c) at least one spacer comprising at least one spacer reactive group, wherein the spacer separates the at least one functional entity from the at least one complementing entity. Each second building block comprises (a) at least one complementing entity comprising a second complementing element comprising at least one recognition group capable of recognising a predetermined second coding element, and (b) at least one spacer comprising at least one spacer reactive group.

Thereafter the coding elements are complemented by contacting each coding element with a building block complementing element capable of recognising the coding element, wherein at least 2 coding elements interact with complementing elements simultaneously, with the proviso that a total of at least 3 first coding elements are complemented. A spacer backbone then is formed by linking neighbouring spacers in a ribosome catalysed reaction by means of reacting spacer reactive groups to produce a templated molecule comprising at least 3 covalently linked, functional groups.

WO 2004/056994 is directed to the synthesis of molecules guided by connector polynucleotides (CPNs) capable of hybridising to complementary connector polynucleotides (CCPNs) harbouring at least one functional entity comprising at least one reactive group. At least one of the CCPNs is capable of hybridising to at least two CPNs. Each CPN is said to "call" for one or more CCPNs capable of hybridisation to the CPN. Following the formation of a supramolecular hybridisation complex comprising a plurality of CPNs and a plurality of CCPNs, the reaction of functional entity reactive groups results in the formation of a molecule comprising covalently linked functional entities. The formation of the molecule involves the transfer of functional entities from one or more "donor CCPNs" to at least one "acceptor CCPN" with which the transferred functional entities were not associated prior to the transfer.

Tamura and Schimmel have reported RNA-templated synthesis to direct peptide bond formation in an order determined by intrinsic differences in substrate reactivity. See, Tamura *et al.* (2003) PROG. NATL. ACAD. SCI. USA 100: 8666-8669. Relying on substrate reactivity differences, however, imposes significant constraints on the order of building blocks within the possible products. Even with precisely tuned reactivities, typical multi-step syntheses still require multiple sequential additions of reactants to form ordered products. See, Zhang et al. (1999) J. AM. CHEM. SOC. 121: 734-753.

Thus, there remains a need for efficient and effective methodologies that allow ordered multi-step synthesis.

### SUMMARY

The present invention is based, in part, upon the discovery that ordered multi-step synthesis can be achieved by nucleic acid-mediated chemistry. For example, ordered multi-step syntheses of both a triolefin and a tripeptide can be achieved using DNA-linked substrates of comparable intrinsic reactivity that are simultaneously present in one solution. These new approaches provide improved yields and efficiency of multi-step products such as synthetic small molecules and synthetic polymers.

In one aspect, the present invention relates to a method of performing multiple sequential nucleic acid-mediated reactions in a single reaction mixture as set forth in claim 1.

In one embodiment, all reactions are mediated by hybridization of the oligonucleotides associated with the reactive units to the template oligonucleotide.

In one embodiment, at least one of the reactions is controlled by the secondary structure of one or more of the oligonucleotides. In another embodiment, all of the reactions is controlled by the secondary structure of one or more of the oligonucleotides.

In one embodiment, the secondary structure is modulated by the reaction conditions, for example, temperature, pH, salt concentration, or a combination of two or more of the foregoing.

In another aspect, the invention provides a method of performing multiple sequential nucleic acid-mediated reactions to produce a reaction product as set forth in claim 4.

In step (b), one or more of the reaction conditions, for example, temperature, can be adjusted, for example, increased, to melt the duplex. Once the duplex forming oligonucleotide has been removed, the remaining template becomes more flexible, for example, portions of the intervening sequence can be looped out, to permit the two reactive units annealed to the template to come into reactive proximity to react with one another and form a product. In this approach, the duplex forming oligonucleotide can be an anti-codon sequence of a transfer unit that anneals to a third codon disposed between the first and second codons.

A library of chemical compounds can be prepared by any of the methods described herein.

Throughout the description, where compositions are described as having, including, or comprising specific components, or where processes are described as having, including, or comprising specific process steps, it is contemplated that compositions of the present invention also consist essentially of, or consist of, the recited components, and that the processes of the present invention also consist essentially of, or consist of, the recited processing steps. Further, it should be understood that the order of steps or order for performing certain actions are immaterial so long as the invention remains operable. Moreover, unless specified to the contrary, two or more steps or actions may be conducted simultaneously.

The foregoing aspects and embodiments of the invention may be more fully understood by reference to the following figures, detailed description and claims. Any methods not within the scope of the invention are discussed for illustration purposes.

### DEFINITIONS

The term, "associated with" as used herein describes the interaction between or among two or more groups, moieties, compounds, monomers, *etc*. When two or more entities are "associated with" one another as described herein, they are linked by a direct or indirect covalent or non-covalent interaction. Preferably, the association is covalent. The covalent association may be, for example, but without limitation, through an amide, ester, carbon-carbon, disulfide, carbamate, ether, thioether, urea, amine, or carbonate linkage. The covalent association may also include a linker moiety, for example, a photocleavable linker. Desirable non-covalent interactions include hydrogen bonding, van der Waals interactions, dipole-dipole interactions, pi stacking interactions, hydrophobic interactions, magnetic interactions, electrostatic interactions, *etc.* Also, two or more entities or agents may be "associated with" one another by being present together in the same composition.

The term, "biological macromolecule" as used herein refers to a polynucleotide (*e*.*g*., RNA, DNA, RNA/DNA hybrid), protein, peptide, lipid, or polysaccharide. The biological macromolecule may be naturally occurring or non-naturally occurring. In a preferred embodiment, a biological macromolecule has a molecular weight greater than about 5,000 Daltons.

The terms, "polynucleotide," "nucleic acid", or "oligonucleotide" as used herein refer to a polymer of nucleotides. The polymer may include, without limitation, natural nucleosides (*i*.*e*., adenosine, thymidine, guanosine, cytidine, uridine, deoxyadenosine, deoxythymidine, deoxyguanosine, and deoxycytidine), nucleoside analogs (*e*.*g*., 2-aminoadenosine, 2-thiothymidine, inosine, pyrrolo-pyrimidine, 3-methyl adenosine, 5-methylcytidine, C5-bromouridine, C5-fluorouridine, C5-iodouridine, C5-propynyl-uridine, C5-propynyl-cytidine, C5-methylcytidine, 7-deazaadenosine, 7-deazaguanosine, 8-oxoadenosine, 8-oxoguanosine, O(6)-methylguanine, and 2-thiocytidine), chemically modified bases, biologically modified bases (*e*.*g*., methylated bases), intercalated bases, modified sugars (*e*.*g*., 2'-fluororibose, ribose, 2'-deoxyribose, arabinose, and hexose), or modified phosphate groups (*e*.*g*., phosphorothioates and 5' -N-phosphoramidite linkages). Nucleic acids and oligonucleotides may also include other polymers of bases having a modified backbone, such as a locked nucleic acid (LNA), a peptide nucleic acid (PNA), a threose nucleic acid (TNA) and any other polymers capable of serving as a template for an amplification reaction using an amplification technique, for example, a polymerase chain reaction, a ligase chain reaction, or non-enzymatic template-directed replication.

The term, "small molecule" as used herein, refers to an organic compound either synthesized in the laboratory or found in nature having a molecular weight less than 10,000 grams per mole, optionally less than 5,000 grams per mole, and optionally less than 2,000 grams per mole.

The terms, "small molecule scaffold" or "molecular scaffold" as used herein, refer to a chemical compound having at least one site or chemical moiety suitable for functionalization. The small molecule scaffold or molecular scaffold may have two, three, four, five or more sites or chemical moieties suitable for functionalization. These functionalization sites may be protected or masked as would be appreciated by one of skill in this art. The sites may also be found on an underlying ring structure or backbone.

The term, "transfer unit" as used herein, refers to a molecule comprising an oligonucleotide having an anti-codon sequence associated with a reactive unit including, for example, but not limited to, a building block, monomer, monomer unit, molecular scaffold, or other reactant useful in template mediated chemical synthesis.

The term, "template" as used herein, refers to a molecule comprising an oligonucleotide having at least one codon sequence suitable for a template mediated chemical synthesis. The template optionally may comprise (i) a plurality of codon sequences, (ii) an amplification means, for example, a PCR primer binding site or a sequence complementary thereto, (iii) a reactive unit associated therewith, (iv) a combination of (i) and (ii), (v) a combination of (i) and (iii), (vi) a combination of (ii) and (iii), or a combination of (i), (ii) and (iii).

The terms, "codon" and "anti-codon" as used herein, refer to complementary oligonucleotide sequences in the template and in the transfer unit, respectively, that permit the transfer unit to anneal to the template during template mediated chemical synthesis.

The term, "oligonucleotide mask" refers to an oligonucleotide sequence complementary to at least a portion of a codon sequence, which, when annealed to the codon sequence, prevents the anti-codon sequence from annealing to the codon sequence under one set of conditions but under a second, different set of conditions is no longer annealed to the codon sequence to permit the anti-codon and codon sequences to anneal to one another.

### BRIEF DESCRIPTION OF THE DRAWINGS

The invention may be further understood from the following figures in which:

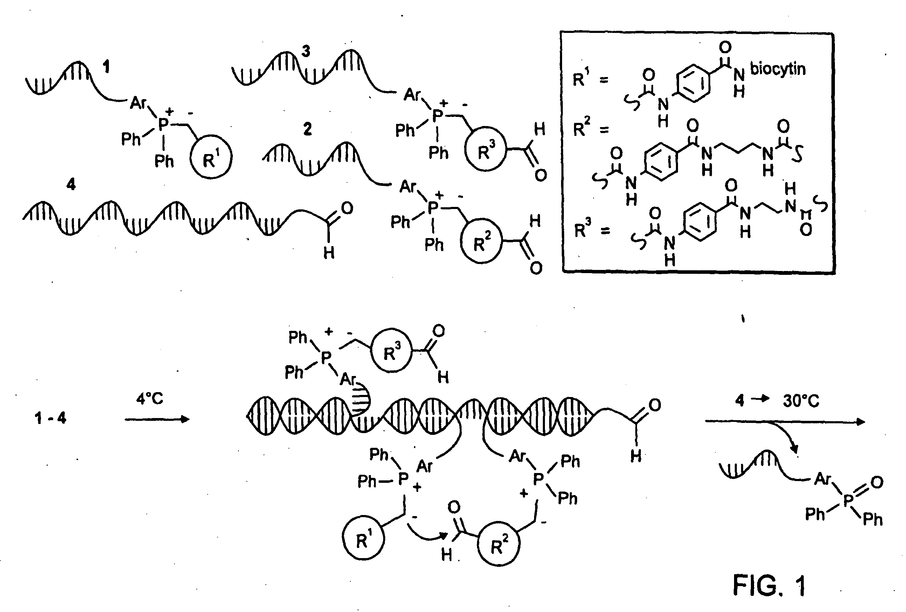

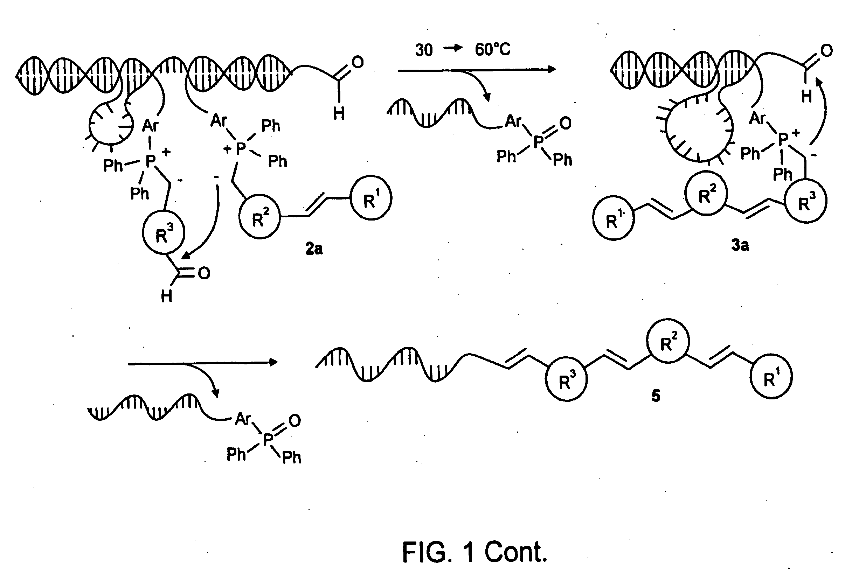

**FIG. 1** is a schematic representation of an exemplary embodiment of an ordered multi-step nucleic acid-mediated chemistry, namely, a strategy for the single-solution synthesis of an ordered triolefin. Building blocks are transferred sequentially among phosphorane reagents **1-3** before addition to an aldehyde-linked template **4.** The rigidity of double-stranded DNA enforces Wittig olefination regioselectivity. As the reaction temperature is elevated, the DNA secondary structure undergoes sequence-programmed changes that enables the desired Wittig olefination to take place selectively.

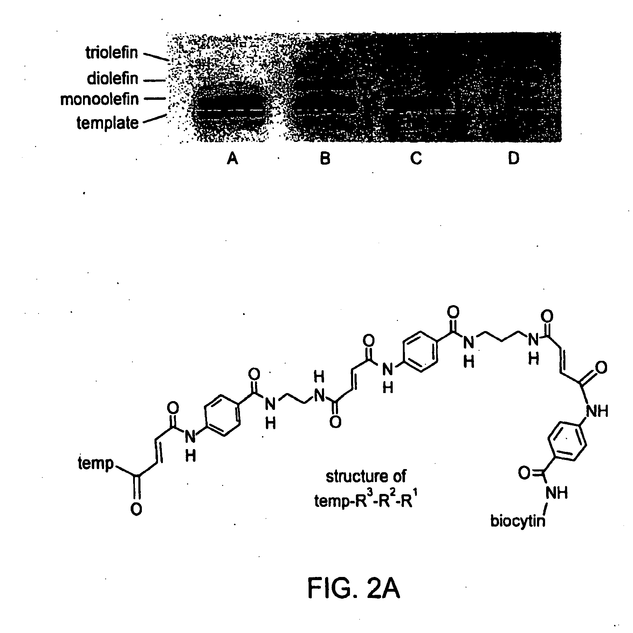

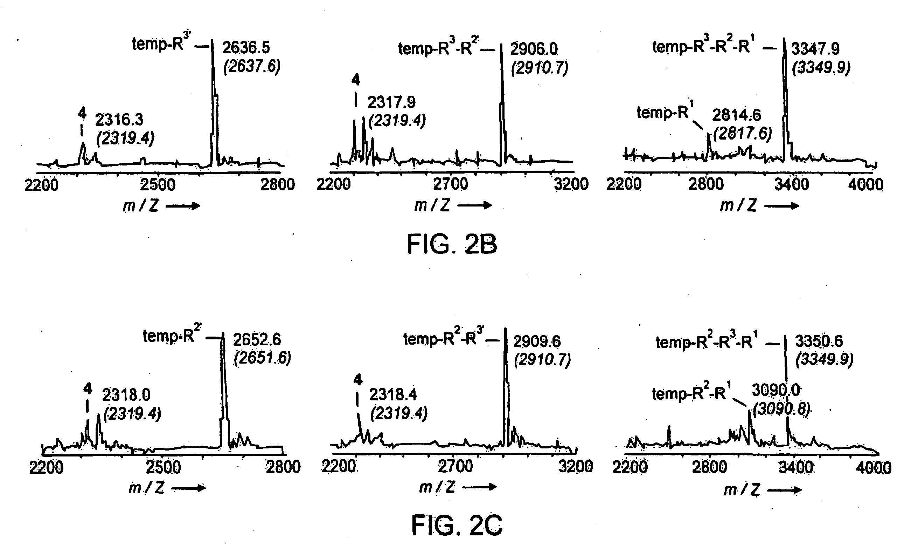

**FIG. 2A** shows a denaturing polyacrylamide gel electrophoresis (PAGE) analysis of the ordered triolefin synthesis. 100 nM of **1-4** were hybridized in aqueous 50 mm NaOAc (pH 5.0), 1 M NaCl; then treated with 0.1 M N-tris(hydroxymethyl)methyl-3-aminopropanesulfonic acid (TAPS; pH 8.0), and 1 M NaCl; and incubated for 1 hour at 48°C, **1** hour at 30°C, and 2 hours at 60°C. The crude reaction mixture is shown in lane C with the streptavidin-captured product in lane D. Control reactions lacking an aldehyde group on both **2** and **3** (lane A) or on **2** only (lane B) were performed under identical conditions to produce either a monoolefin or diolefin, respectively. **FIG. 2B** depicts MALDI-TOF mass spectroscopic data of products from reactions shown in **FIG. 2A**. The three spectra correspond, from left to right, to lanes A, B, and D from **FIG. 2A**, respectively. **FIG. 2C** depicts MALDI-TOF mass-spectroscopic data for the reactions using swapped building blocks (R³ attached to **2**, and R² attached to 3). Expected masses for samples in **FIG. 2B** and **FIG. 2C** are listed in parentheses; the expected error is 6 Da. The prime designation (R^{2,} and R^{3,}) in **FIG. 2B** and **FIG. 2C** refers to the forms of these building blocks lacking aldehyde groups, and temp indicates template **4**.

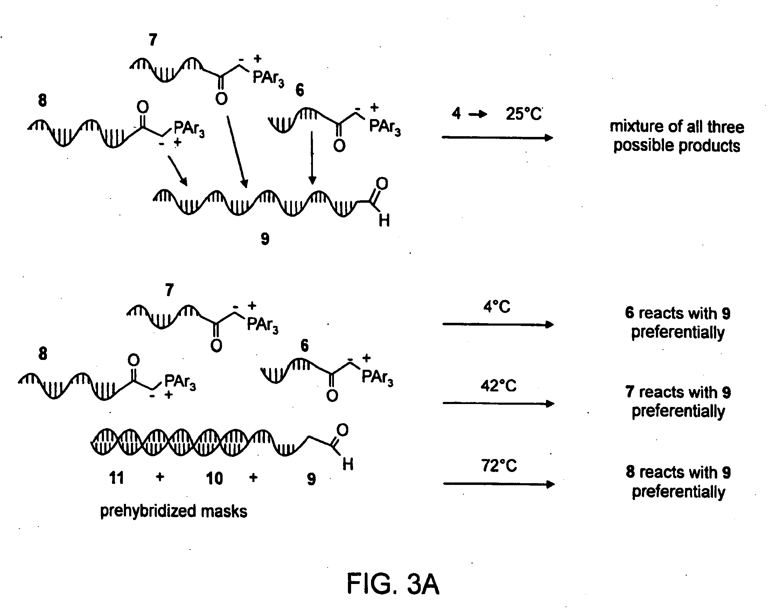

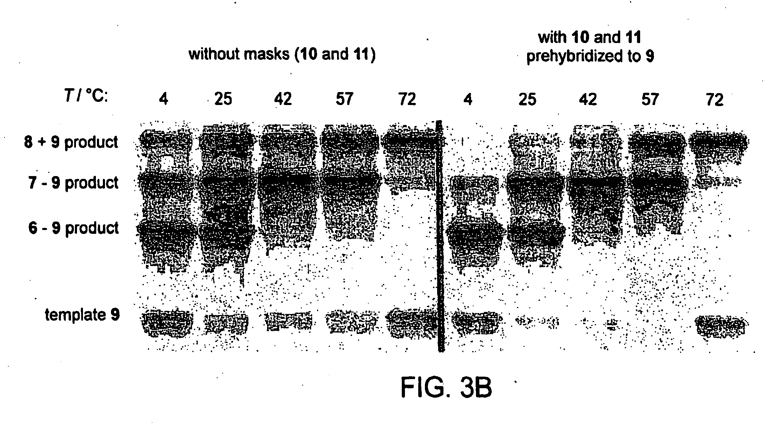

**FIG. 3A** is a schematic representation of an exemplary scheme of ordered multi-step nucleic acid-mediated synthesis, namely, a strategy for using oligonucleotide masks **10** and **11** to order the reaction of three reagents **6-8** with a template **9.** When all masks are hybridized (4°C), only **6** can react. At an intermediate temperature (42°C), mask **10** is melted from the template allowing **7** to react exclusively. At a high temperature (72°C), only **8** can react. **FIG. 3B** is a denaturing PAGE gel of the reaction products from **FIG. 3A**. 150 nm of **9** (with or without masks **10** and **11** at 225 nm) was incubated at the indicated temperature. Reagents **6-8** were added simultaneously to 200 nM each and the reaction was incubated 1 hour before analysis.

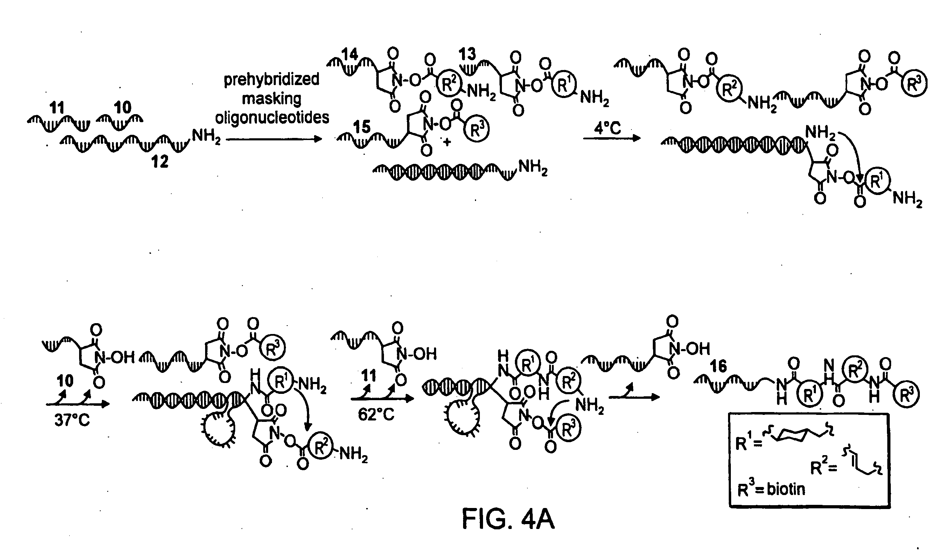

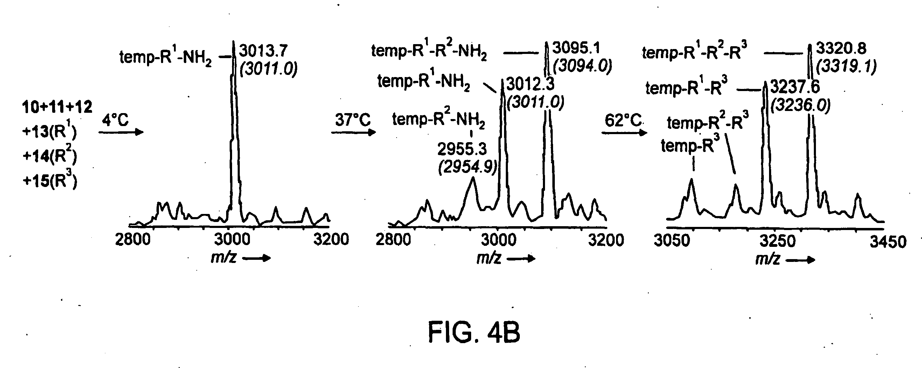

**FIG. 4A** is a schematic representation of an exemplary scheme of ordered multi-step nucleic acid-mediated synthesis, namely, a strategy for single-solution synthesis of an ordered tripeptide using oligonucleotide masks. **FIG. 4B** shows MALDI-TOF mass spectroscopy data of each stage of the reaction in **FIG. 4A**. 200 nM **of 12** was prehybridized to 1.5 equivalents each of **10** and **11** in 0.2 M 3-(N-morpholino)-propanesulfonic acid (MOPS, pH 7.0), 2 M NaCl at 48°C. Simultaneously, 1.05 equivalents of **13** and **14** and 3.0 equivalents of **15** were added to **10+11+12.** After dilution caused by the addition of the reagents, the final concentration of solutes in this reaction mixture was 0.1 M MOPS (pH 7.0), 1 M NaCl with 100 nM template **12.** The reaction mixture was incubated at 20 minutes at 4°C, 20 minutes at 37°C, and 2 hours at 62°C. Reactions were quenched with Tris (after either of the first two steps) or purified with streptavidin-linked beads (after the third step) before analysis. Expected masses for samples in **FIG. 4B** are listed in parentheses; the expected error is 6 Da, and temp indicates template **12.**

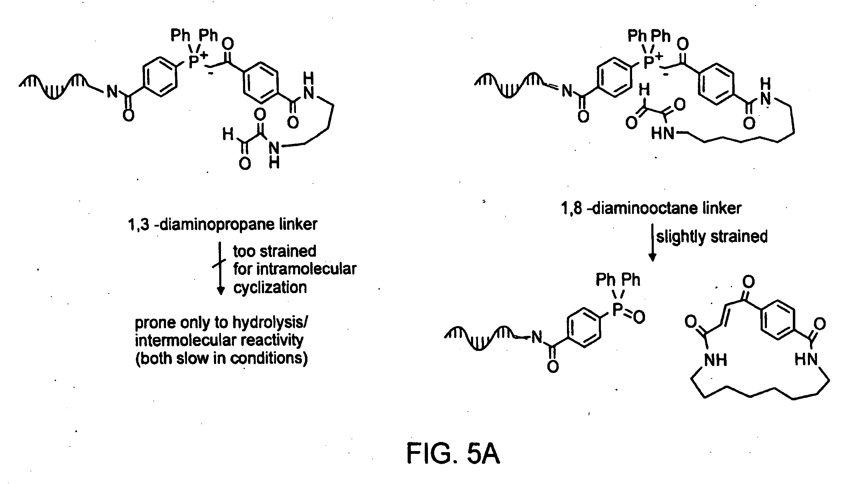

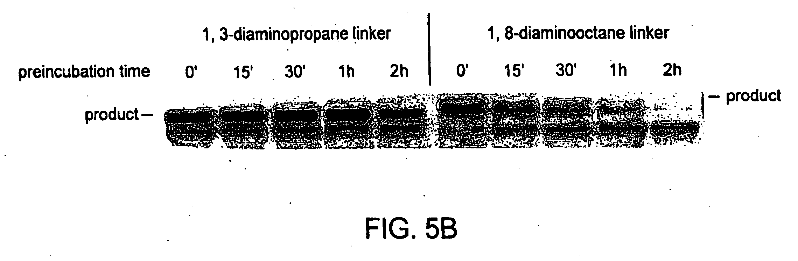

**FIG. 5A** is a schematic representation of an exemplary embodiment of stability analysis of phosporane reagents. **FIG. 5B** shows the corresponding PAGE analysis. An intramolecular cyclization is only possible for the long octane linker and the reactivity of this reagent noticeably decreases with preincubation in pH 8.0 buffer; the reagent with the shorter propane linker maintains most of its reactivity even after 2 hours at 25 °C.

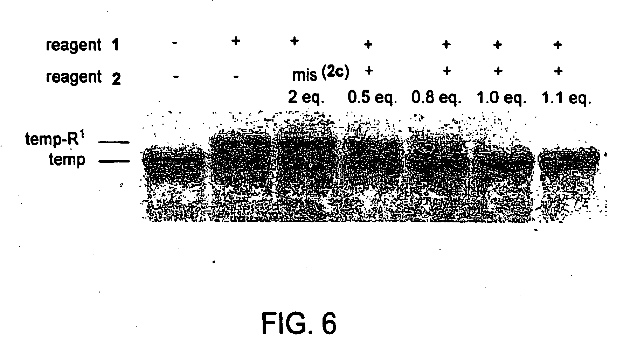

**FIG. 6** shows the PAGE analysis of the reaction of 1 directly to **4,** in the presence and absence of **2.** While the transfer of R¹ to the template **4** is observed in the absence of **2** and with insufficient equivalents of **2,** once 1.0 equivalent of **2** relative to **4** are present, the direct transfer of R¹ to **4** is no longer observed under the multistep reaction conditions (1 hour at 4 °C, 1 hour at 30 °C, 2 hours at 60 °C).

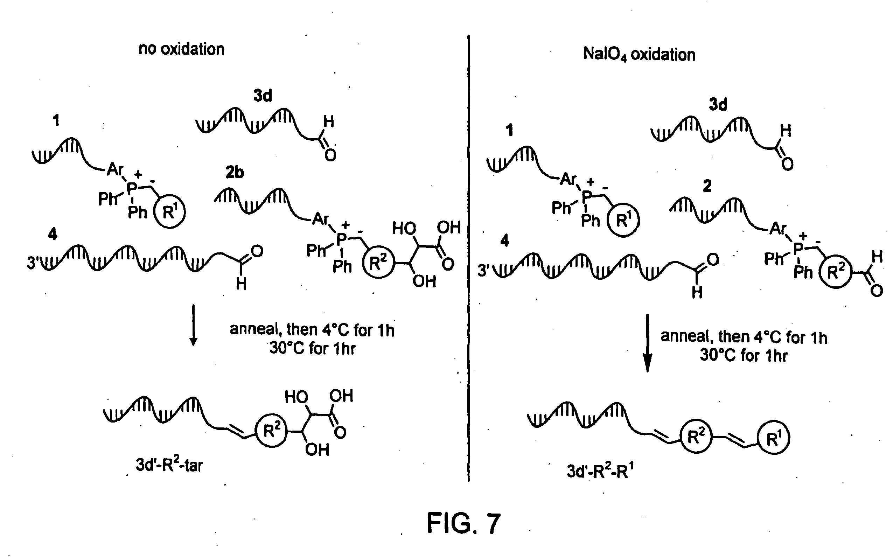

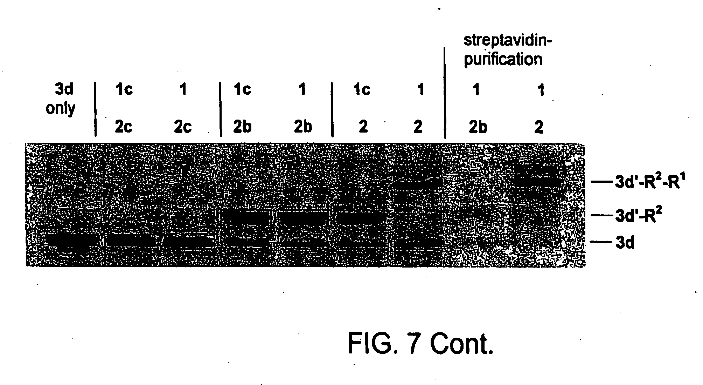

**FIG. 7** is a schematic representation of an exemplary embodiment of a two-step Wittig olefination sequence. As shown schematically, reactions were carried out with matched **(1, 2/2b)** or mismatched **(1c/2c)** reagents as well as a modified reagent **3d** that can stably capture any intermediates that react with it. The only biotinylated products result from the use of oxidized (aldehyde containing) and sequence-matched reagents **(1** and **2).** Without oxidation, only the tartrate on **2b** can be transferred to **3d.** The prime notation on **3d'** in the product labels indicates that the aldehyde on **3d** has reacted to form an alkene.

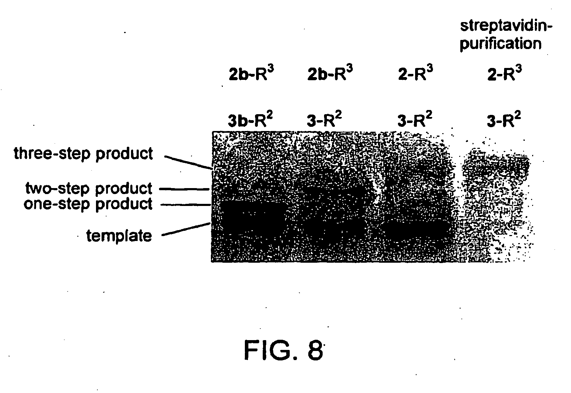

**FIG. 8** shows the PAGE analysis of an exemplary embodiment of an ordered triolefin synthesis to generate the product, similar to **FIG. 2A****,** but using reagents with switched building blocks.

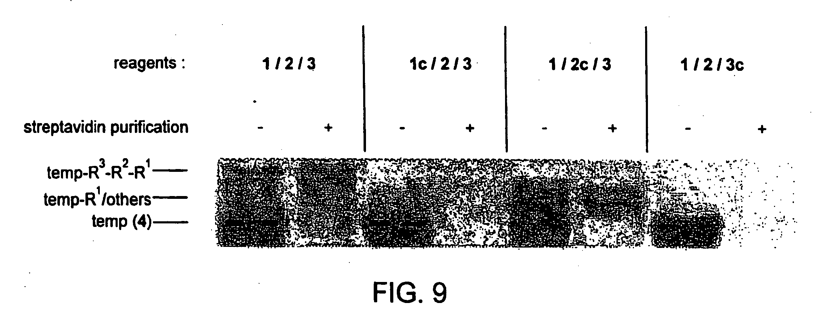

**FIG. 9** shows the PAGE analysis of an exemplary embodiment including mismatch controls of a three-step sequence. The matched three-step sequence is shown in the two left most lanes with the remaining lanes containing the three mismatch controls. For the mismatch controls, only with mismatched reagent **2c** is any biotinylated material transferred to the template, and this product corresponds to R¹ adding directly. Using mismatch reagents **1c** or **3c** lead to no modified templates.

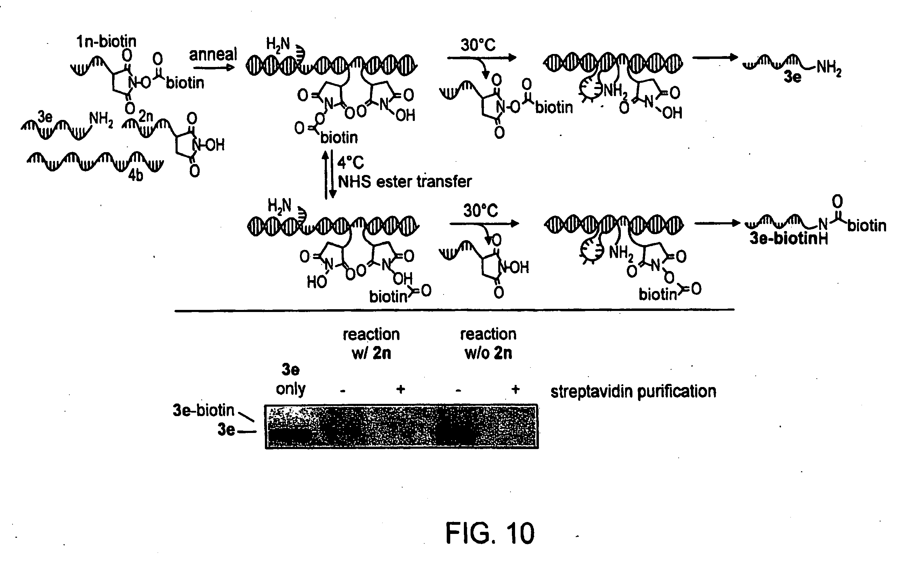

**FIG. 10** is a schematic representation of an exemplary embodiment demonstrating transfer of an NHS ester onto DNA-linked NHS. Because of the potential for the NHS group to serve as a nucleophile and attack an NHS ester, the ordered synthesis of a tripeptide cannot be performed efficiently using this transfer scheme. The reaction with NHS-linked DNA **2n** can transfer biotin from **1n** to **3e** but no transfer is seen when **2n** is excluded.

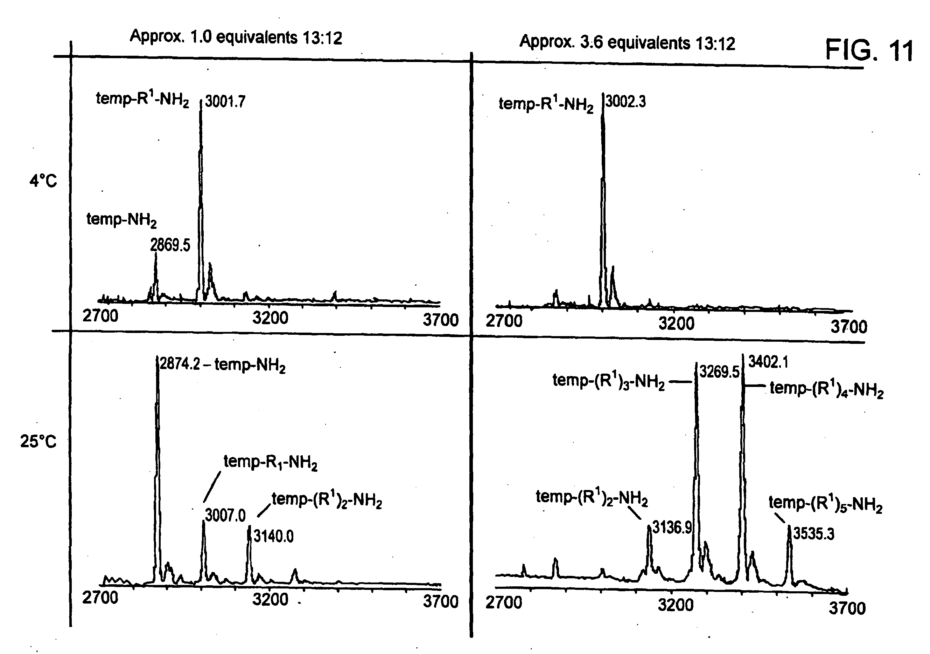

**FIG. 11** shows the reactivity of NHS-linked amino acid reagents as analyzed by MALDI-TOF, for an exemplary embodiment. Different numbers of equivalents of **13** were added to **12** (with **10** and **11**) at either 4 °C or 25 °C. At temperatures near the melting temperature of the reagent, exchange of the oligonucleotides leads to multiple additions of a single reagent to the template. At temperatures much lower than Tm, the reagent, once hybridized, remains in a stable duplex.

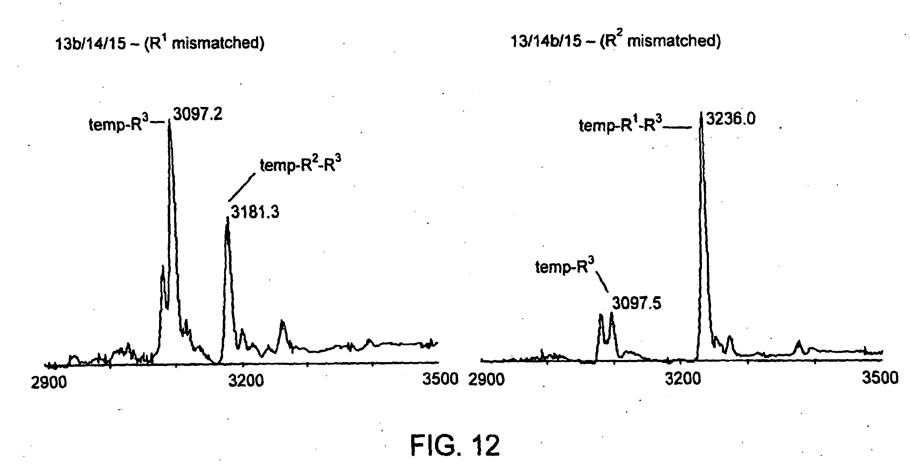

**FIG. 12** shows MALDI-TOF results of an exemplary embodiment of three-step reactions with mismatched reagents, showing that no incorporation of the building block on the mismatched reagent is detected for either reaction.

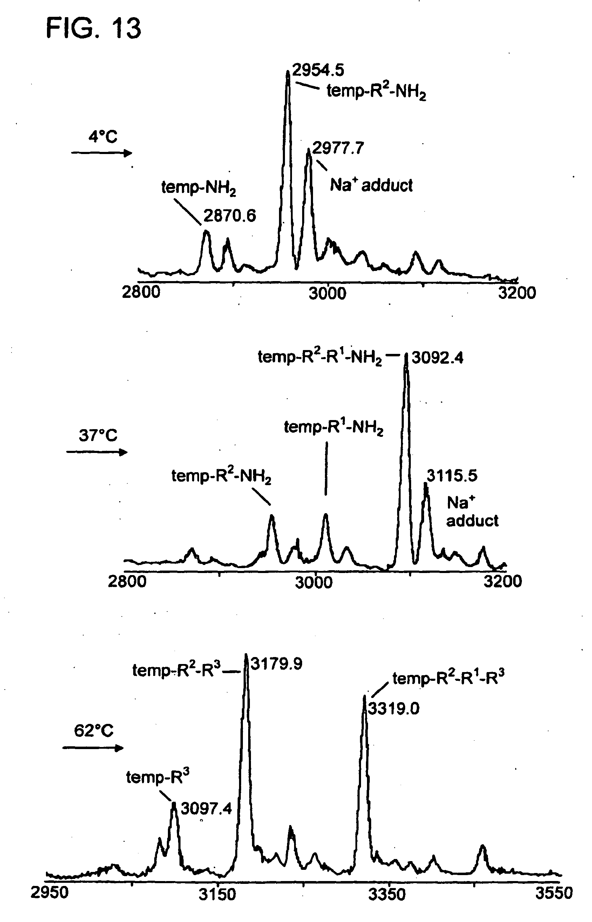

**FIG. 13** shows MALDI-TOF results of an exemplary embodiment of a three step sequence with certain building blocks switched. Building blocks on **13** and **14** are swapped. Just as with the sequence shown in **FIG. 2B****,** the building block on **13** (in this case R²) adds first at 4 °C and then the building block on **14** (in this case R¹) adds at 37 °C. Differences in MALDI ionization may lead to the lower overall signal for the tripeptide product relative to the truncated dipeptide for the final product mixture.

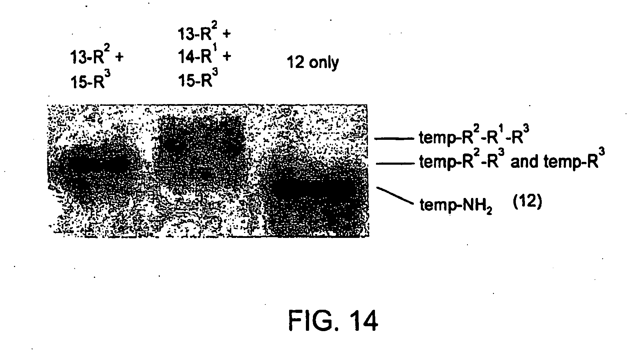

**FIG. 14** shows the page analysis of an exemplary embodiment of an R²-R¹-R³ tripeptide sequence. While the reaction that excludes the second reagent (**14**-R¹) runs as a single band, containing both temp-R²-R³ and temp-R³, the reaction with all three reagents runs as two bands with the upper band representing the tripeptide product with R¹. Based on densitometry analysis, the tripeptide represents 55% of the products in the final isolated mixture.

### DESCRIPTION OF THE INVENTION

The present invention is based in part on the surprising discovery of ordered multi-step syntheses by nucleic acid-mediated chemistry. More particularly, it is discovered that ordered multi-step syntheses of, for example, a triolefin and a tripeptide can be achieved using DNA-linked substrates of comparable intrinsic reactivity that are simultaneously present in one solution. In both cases, reaction conditions, for example, temperature-sensitive variations in DNA secondary structure orchestrate a series of effective molarity changes among different reactants to preferentially generate one ordered product out of many possibilities. This biomimetic approach to ordering a chemical synthesis produces increased yields of multi-step products and facilitates the application of evolutionary principles to the selection of functional synthetic small molecules and synthetic polymers.

These results described herein represent two exemplary strategies for ordered synthesis without the structural constraints imposed by enzymes and significantly enhance the efficiency and selectivity of multi-step DNA-templated synthesis. For a discussion on nucleic acid-mediated chemistry, see, e.g., Gartner et al. (2002) J. AM. CHEM. Soc. 124: 10304-10306; Gartner et al. (2004) SCIENCE 305(10): 1601-1605; Liu et al.(2002) ANGEW. CHEM. IN. ED. 41(10): 1796-2000; U.S. Patent Application Publication Nos. 2004/0180412 A1 (10/643,752 Aug. 19, 2003) by Liu et al. and 2003/0113738 A1 (USSN 10/101,030 Mar. 19, 2002) by Liu et al.

The first strategy **(****FIG. 1****)** passes a growing molecule from site-to-site along a template in a manner controlled by DNA secondary structure. Three Wittig olefination substrates (**1-3,** with **1** containing a biotin group), each linked to DNA oligonucleotides of varying melting temperature (Tₘ), were hybridized to an aldehyde-terminated DNA template **4** at 4 °C. Gartner et al. (2002) Angew. Chem. Int. Ed. 123: 61796-1800; Gartner et al. (2003) ANGEW. CHEM. INT. ED. 42: 1370-1375.

If these substrates were combined at the high concentrations (mM-M) common to organic synthesis, a complex mixture of many products would result from their random reaction. At a concentration of 100 nM each, however, only substrates juxtaposed in a productive way by DNA hybridization can react at a significant rate. (Gartner et al. (2001) J. AM. CHEM. SOC. 123: 6961-6963.)

Upon phosphonium deprotonation, seven intermolecular Wittig olefinations were in principle possible among these four reactants. At the lowest temperature (4 °C), however, six of the seven possible Wittig reactions are precluded because their reactants are separated by double-stranded DNA. Previous studies have shown that double-stranded DNA's rigidity can enforce the separation of substrates that flank duplex DNA, thus preventing their reaction (Gartner et al. (2001), J. AM. CHEM. SOC. 123: 6961-6963). At 4 °C, the only phosphorane-aldehyde not separated by duplex DNA, and therefore able to undergo Wittig olefination, are **1** and **2** which react to generate DNA-linked monoolefin **2a.**

As the temperature was elevated to 30 °C, the reagent with the lowest Tₘ (the phosphine oxide of **1)** dissociated from the template allowing reactants **2a** and **3**, no longer separated by double-stranded DNA, to react selectively to form diolefin **3a.** When the temperature was increased to 60 °C for 2 hours, the phosphine oxide of **2** dissociated, which enabled the final reaction to take place between **4** and **3a,** generating ordered triolefin **5 (****FIG. 1****).**

Following the capture of R¹-linked products using streptavidin-agarose beads, triolefin **5** was obtained at a yield of 24% **(****FIG. 2A****,** lanes C and D). As expected from the biotin-based purification method, products lacking R¹ were not detected. Truncated products represented less than 10% of the isolated material as analyzed by denaturing PAGE analysis. To confirm the order of the building blocks in the products, control reactions were performed with reactants lacking aldehyde groups. Removing the aldehyde group from **2** prevented the reaction of **1** with **2** and resulted in a diolefin template R³-R^{2,} product **(****FIG. 2A****,** lane B). Removing the aldehyde group from both 3 and 2 prevented all reactions except for the reaction of **3** with template 4 to generate the monoolefin template R³'. MALDI-TOF analysis was consistent with the expected product structures **(****FIG. 2B****).**

Ordered triolefin syntheses and control reactions were repeated using a different set of reagents in which R² and R³ were interchanged on **2** and **3.** The resulting triolefin product and control truncated mono/di-olefins exhibited the expected R²-R³-R¹ order of building blocks (See Example 1). Additional control reactions using sequence-mismatched reagents **1c, 2c**, or **3c** instead of **1, 2,** or **3** resulted in either no transfer of biotinylated R¹ to **4** (using **1c** or **3c**), or in the direct reaction of **1** and **4** (using **2c**; See Example 1). Collectively, these results demonstrate that the order of Wittig olefination in this system is tightly controlled by sequence-programmed changes in DNA secondary structure and not by intrinsic reactivity differences among substrates.

The synthesis of an ordered tripeptide from a single solution containing three N-hydroxysuccinimidyl (NHS) ester-activated amino acids required a different strategy. While the Wittig reaction is irreversible, amine acylation with an NHS ester generates a nucleophilic NHS group capable of re-capturing and modifying products before dissociating from the template. An alternate approach for ordered peptide synthesis was developed **(****FIG. 3A****).** Although a mixture of all possible products was formed when DNA-linked phosphoranes **6-8** were added simultaneously to aldehyde-linked template, **9,** when masks **10** and **11** were prehybridized to the template before the addition of **6-8,** reactivity became strongly dependent on the reaction temperature approaching or exceeding the Tₘ value of each mask (25-35 °C for **10** and 60-65 °C for **11,** **FIG. 3B**).

This approach allowed for a single-solution ordered tripeptide synthesis **(****FIG. 4A****).** An amine-terminated template **12** was prehybridized with oligonucleotide masks **10** and **11** at 4 °C. When NHS ester linked reagents **13-15** were combined with the masked template **10+11+12** at 4 °C for 20 minutes, only **13** could hybridize to the template and react to generate a monopeptide. As the solution was heated to 37 °C for 20 minutes, both the first reagent and the first mask **10** dissociated from the template, thus exposing the binding site for the second reagent **14** which then hybridized and reacted to generate a template-linked dipeptide. At the highest temperature (62 °C for 2 hours), all masks and reagents were melted except for **15,** which hybridized and reacted to form ordered tripeptide **16.** Unlike the first strategy in which incomplete reactivity reduces overall yield but does not generate truncated biotinylated byproducts, this strategy requires each step to proceed in high yield to generate the tripeptide (and not truncated mono/di-peptides) as the major product.

Aliquots of the reaction taken after the 4° C and 37° C incubations were quenched by the addition of 1 M tris(hydroxymethyl)aminomethane (Tris). Analysis by MALDI-TOF mass spectrometry **(****FIG. 4B****)** indicated that **13** exclusively reacts with the template at 4 °C, followed by the reaction of **14** at 37 °C. Biotinylated templates arising from the reaction of **15** were isolated using streptavidin-linked beads in 45% yield as determined by denaturing PAGE and analyzed by MALDI-TOF **(****FIG. 4B****).** The strongest signal in the purified product mixture is the desired tripeptide product **16** with the most significant side product being the truncated dipeptide R¹-R³ which results from the lack of reaction with **14.** Sequence-mismatched reagents **13b** or **14b** were unable to couple in place of **13** or **14** (see Example 1).

When the oligonucleotides linked to R¹ and R² were swapped, the order of building block addition was also switched (R² added first at 4 °C, then R¹ added at 37 °C, See Example 1). The biotinylated products of this reaction sequence, including the ordered tripeptide **12**-R²-R¹-R³, were isolated in 38% yield after streptavidin purification. For this reaction, the desired tripeptide and truncated side products were resolvable by denaturing PAGE (unlike the case of **16),** revealing that 55% of the isolated material (21% total overall yield) is the ordered tripeptide (See Example 1). These results collectively indicate that the use of temperature-controlled template masking enables substrates that would normally form a vast mixture of oligomeric products to react in a predominantly ordered manner. As in the triolefin example, the above findings indicate that DNA sequences, rather than reactivity differences among substrates, dictate the order of building blocks within the resulting tripeptide product.

These results represent single-solution ordered multi-step syntheses using comparably reactive substrates in the absence of enzymes with only a temperature gradient needed to coordinate the timing of the three successive reactions. Both strategies offer faster, higher-yielding routes to multi-step DNA-linked products than in the past.

### I. TEMPLATE CONSIDERATIONS

The nucleic acid template can direct a wide variety of chemical reactions without obvious structural requirements by sequence-specifically recruiting reactants linked to complementary oligonucleotides. As discussed, the nucleic acid-mediated format permits reactions that may not be possible using conventional synthetic approaches. During synthesis, the template hybridizes or anneals to one or more transfer units to direct the synthesis of a reaction product, which during certain steps of templated synthesis remain associated with the template. A reaction product then is selected or screened based on certain criteria, such as the ability to bind to a preselected target molecule. Once the reaction product has been identified, the associated template can then be sequenced to decode the synthetic history of the reaction product. Furthermore, as will be discussed in more detail below, the template may be evolved to guide the synthesis of another chemical compound or library of chemical compounds.

### (i) Template Format

The template may incorporate a hairpin loop on one end terminating in a reactive unit that can interact with one or more reactive units associated with transfer units. For example, a DNA template can comprise a hairpin loop terminating in a 5'-amino group, which may or may not be protected. The amino group may act as an initiation point for formation of an unnatural polymer or small molecule.

The length of the template may vary greatly depending upon the type of the nucleic acid-templated synthesis contemplated. For example, in certain embodiments, the template may be from 10 to 10,000 nucleotides in length, from 20 to 1,000 nucleotides in length, from 20 to 400 nucleotides in length, from 40 to 1,000 nucleotides in length, or from 40 to 400 nucleotides in length. The length of the template will of course depend on, for example, the length of the codons, the complexity of the library, the complexity and/or size of a reaction product, the use of spacer sequences, *etc.*

### (ii) Codon Usage

It is contemplated that the sequence of the template may be designed in a number of ways without going beyond the scope of the present invention. For example, the length of the codon must be determined and the codon sequences must be set. If a codon length of two is used, then using the four naturally occurring bases only 16 possible combinations are available to be used in encoding the library. If the length of the codon is increased to three (the number Nature uses in encoding proteins), the number of possible combinations increases to 64. If the length of the codon is increased to four, the number of possible combinations increases to 256. Other factors to be considered in determining the length of the codon are mismatching, frame-shifting, complexity of library, *etc.* As the length of the codon is increased up to a certain point the number of mismatches is decreased; however, excessively long codons likely will hybridize despite mismatched base pairs.

Although the length of the codons may vary, the codons may range from 2 to 50 nucleotides, from 2 to 40 nucleotides, from 2 to 30 nucleotides, from 2 to 20 nucleotides, from 2 to 15 nucleotides, from 2 to 10 nucleotides, from 3 to 50 nucleotides, from 3 to 40 nucleotides, from 3 to 30 nucleotides, from 3 to 20 nucleotides, from 3 to 15 nucleotides, from 3 to 10 nucleotides, from 4 to 50 nucleotides, from 4 to 40 nucleotides, from 4 to 30 nucleotides, from 4 to 20 nucleotides, from 4 to 15 nucleotides, from 4 to 10 nucleotides, from 5 to 50 nucleotides, from 5 to 40 nucleotides, from 5 to 30 nucleotides, from 5 to 20 nucleotides, from 5 to 15 nucleotides, from 5 to 10 nucleotides, from 6 to 50 nucleotides, from 6 to 40 nucleotides, from 6 to 30 nucleotides, from 6 to 20 nucleotides, from 6 to 15 nucleotides, from 6 to 10 nucleotides, from 7 to 50 nucleotides, from 7 to 40 nucleotides, from 7 to 30 nucleotides, from 7 to 20 nucleotides, from 7 to 15 nucleotides, from 7 to 10 nucleotides, from 8 to 50 nucleotides, from 8 to 40 nucleotides, from 8 to 30 nucleotides, from 8 to 20 nucleotides, from 8 to 15 nucleotides, from 8 to 10 nucleotides, from 9 to 50 nucleotides, from 9 to 40 nucleotides, from 9 to 30 nucleotides, from 9 to 20 nucleotides, from 9 to 15 nucleotides, from 9 to 10 nucleotides. Codons, however, preferably are 3, 4, 5, 6, 7, 8, 9 or 10 nucleotides in length.

In one embodiment, the set of codons used in the template maximizes the number of mismatches between any two codons within a codon set to ensure that only the proper anti-codons of the transfer units anneal to the codon sites of the template. Furthermore, it is important that the template has mismatches between all the members of one codon set and all the codons of a different codon set to ensure that the anti-codons do not inadvertently bind to the wrong codon set. For example, with regard to the choice of codons *n* bases in length, each of the codons within a particular codon set should differ with one another by *k* mismatches, and all of the codons in one codon set should differ by *m* mismatches with all of the codons in the other codon set. Exemplary values for *n*, *k,* and *m*, for a variety of codon sets suitable for use on a template are published, for example, in Table 1 of U.S. Patent Application Publication No. US-2004/0180412, by Liu et al.

Using an appropriate algorithm, it is possible to generate sets of codons that maximize mismatches between any two codons within the same set, where the codons are *n* bases long having at least *k* mismatches between any two codons. Since between any two codons, there must be at least *k* mismatches, any two subcodons of *n* - (*k*-1) bases must have at least one mismatch. This sets an upper limit of 4^{*n-k*+1} on the size of any (*n*, *k*) codon set. Such an algorithm preferably starts with the 4^{*n-k*+1} possible subcodons of length *n* - (*k* - 1) and then tests all combinations of adding *k -* 1 bases for those that always maintain *k* mismatches. All possible (*n*, *k*) sets can be generated for *n* ≤ 6. For *n* > 6, the 4^{*n*-*k*+1} upper limits of codons cannot be met and a "full" packing of viable codons is mathematically impossible. In addition to there being at least one mismatch *k* between codons within the same codon set, there should also be at least one mismatch *m* between all the codons of one codon set and all the codons of another codon set. Using this approach, different sets of codons can be generated so that no codons are repeated.

By way of example, four (*n*=5, *k*=3, *m*=1) sets, each with 64 codons, can be chosen that always have at least one mismatch between any two codons in different sets and at least three mismatches between codons in the same set, as described, for example, in Tables 2-5 of U.S. Patent Application Publication No. US-2004/0180412, by Liu et al.. Similarly, four (*n*=6, *k*=4, *m*=2) sets, each with 64 codons, can be chosen that always have at least two mismatches between any two codons in different codon sets and at least four mismatches between codons in the same codon set as described, for example, in Tables 6-9 of U.S. Patent Application Publication No. US-2004/0180412, by Liu et al.

Codons can also be chosen to increase control over the GC content and, therefore, the melting temperature of the codon and anti-codon. Codons sets with a wide range in GC content versus AT content may result in reagents that anneal with different efficiencies due to different melting temperatures. By screening for GC content among different (*n, k*) sets, the GC content for the codon sets can be optimized. For example, the four (6, 4, 2) codon sets set forth in Tables 6-9 each contain 40 codons with identical GC content (*i*.*e.*, 50% GC content). By using only these 40 codons at each position, all the reagents in theory will have comparable melting temperatures, removing potential biases in annealing that might otherwise affect library synthesis. Longer codons that maintain a large number of mismatches such as those appropriate for certain applications such as the reaction discovery system can also be chosen using this approach. For example, by combining two (6, 4) sets together while matching low GC to high GC codons, (12, 8) sets with 64 codons all with 50% GC content can be generated for use in reaction discovery selections as well as other application where multiple mismatches might be advantageous. These codons satisfy the requirements for encoding a 30 x 30 matrix of functional group combinations for reaction discovery.

Although an anti-codon is intended to bind only to a codon, an anti-codon may also bind to an unintended sequence on a template if complementary sequence is present. Thus, an anti-codon may inadvertently bind to a non-codon sequence. Alternatively, an anti-codon might inadvertently bind out-of-frame by annealing in part to one codon and in part to another codon or to a non-codon sequence. Finally, an anti-codon might bind in-frame to an incorrect codon, an issue addressed by the codon sets described above by requiring at least one base difference distinguishing each codon. In Nature, the problems of noncoding sequences and out-of-frame binding are avoided by the ribosome. The nucleic acid-templated methods described herein, however, do not take advantage of the ribosome's fidelity. Therefore, in order to avoid erroneous annealing, the templates can be designed such that sequences complementary to anti-codons are found exclusively at in-frame codon positions. For example, codons can be designed to begin, or end, with a particular base (*e*.*g*., "G"). If that base is omitted from all other positions in the template (*i*.*e*., all other positions are restricted to T, C, and A), only perfect codon sequences in the template will be at the in-frame codon sequences. Similarly, the codon may be designed to be sufficiently long such that its sequence is unique and does not appear elsewhere in a template.

When the nucleic acid-templated synthesis is used to produce a polymer or a small molecule, spacer sequences may also be placed between the codons to prevent frame shifting. For example, the bases of the template that encode a polymer subunit (the "genetic code" for the polymer) may be chosen from **Table 1** to preclude or minimize the possibility of out-of-frame annealing. These genetic codes reduce undesired frameshifted nucleic acid-templated polymer translation and differ in the range of expected melting temperatures and in the minimum number of mismatches that result during out-of-frame annealing.

**TABLE 1: Representative Genetic Codes for Nucleic Acid-templated Polymers That Preclude Out-Of-Frame Annealing**

| Sequence | Number of Possible Codons |
|---|---|
| VVNT | 36 possible codons |
| NVVT | 36 possible codons |
| SSWT | 8 possible codons |
| SSST | 8 possible codons |
| SSNT | 16 possible codons |
| VNVNT or NVNVT | 144 possible codons |
| SSSWT or SSWST | 16 possible codons |
| SNSNT or NSNST | 64 possible codons |
| SSNWT or SWNST | 32 possible codons |
| WSNST or NSWST | 32 possible codons |

where, V = A, G, or G, S= C or G, W = A or T, and N = A, C, G, or T

As in Nature, start and stop codons are useful, particularly in the context of polymer synthesis, to restrict erroneous anti-codon annealing to non-codons and to prevent excessive extension of a growing polymer. For example, a start codon can anneal to a transfer unit bearing a small molecule scaffold or a start monomer unit for use in polymer synthesis; the start monomer unit can be masked by a photolabile protecting group. A stop codon, if used to terminate polymer synthesis, should not conflict with any other codons used in the synthesis and should be of the same general format as the other codons. Generally, a stop codon can encode a monomer unit that terminates polymerization by not providing a reactive group for further attachment. For example, a stop monomer unit may contain a blocked reactive group such as an acetamide rather than a primary amine. In other embodiments, the stop monomer unit can include a biotinylated terminus that terminates the polymerization and facilitates purification of the resulting polymer.

### (iii) Template Synthesis

The templates may be synthesized using methodologies well known in the art. For example, the nucleic acid sequence may be prepared using any method known in the art to prepare nucleic acid sequences. These methods include both *in vivo* and *in vitro* methods including PCR, plasmid preparation, endonuclease digestion, solid phase synthesis (for example, using an automated synthesizer), *in vitro* transcription, strand separation, *etc.* Following synthesis, the template, when desired may be associated (for example, covalently or non covalently coupled) with a reactive unit of interest using standard coupling chemistries known in the art.

An efficient method to synthesize a large variety of templates is to use a "split-pool" technique. The oligonucleotides are synthesized using standard 3' to 5' chemistries. First, the constant 3' end is synthesized. This is then split into *n* different vessels, where *n* is the number of different codons to appear at that position in the template. For each vessel, one of the *n* different codons is synthesized on the (growing) 5' end of the constant 3' end. Thus, each vessel contains, from 5' to 3', a different codon attached to a constant 3' end. The *n* vessels are then pooled, so that a single vessel contains *n* different codons attached to the constant 3' end. Any constant bases adjacent the 5' end of the codon are now synthesized. The pool then is split into *m* different vessels, where *m* is the number of different codons to appear at the next (more 5') position of the template. A different codon is synthesized (at the 5' end of the growing oligonucleotide) in each of the *m* vessels. The resulting oligonucleotides are pooled in a single vessel. Splitting, synthesizing, and pooling are repeated as required to synthesize all codons and constant regions in the oligonucleotides.

### II. TRANSFER UNITS

A transfer unit comprises an oligonucleotide containing an anti-codon sequence and a reactive unit. The anti-codons are designed to be complementary to the codons present in the template. Accordingly, the sequences used in the template and the codon lengths should be considered when designing the anti-codons. Any molecule complementary to a codon used in the template may be used, including natural or non-natural nucleotides. In certain embodiments, the codons include one or more bases found in nature (*i*.*e*., thymidine, uracil, guanidine, cytosine, and adenine). Thus, the anti-codon can include one or more nucleotides normally found in Nature with a base, a sugar, and an optional phosphate group. Alternatively, the bases may be connected via a backbone other than the sugar-phosphate backbone normally found in Nature (*e*.*g*., non-natural nucleotides).

As discussed above, the anti-codon is associated with a particular type of reactive unit to form a transfer unit. The reactive unit may represent a distinct entity or may be part of the functionality of the anti-codon unit. In certain embodiments, each anti-codon sequence is associated with one monomer type. For example, the anti-codon sequence ATTAG may be associated with a carbamate residue with an isobutyl side chain, and the anti-codon sequence CATAG may be associated with a carbamate residue with a phenyl side chain. This one-for-one mapping of anti-codon to monomer units allows the decoding of any polymer of the library by sequencing the nucleic acid template used in the synthesis and allows synthesis of the same polymer or a related polymer by knowing the sequence of the original polymer. By changing (*e*.*g*., mutating) the sequence of the template, different monomer units may be introduced, thereby allowing the synthesis of related polymers, which can subsequently be selected and evolved. In certain preferred embodiments, several anti-codons may code for one monomer unit as is the case in Nature.

In certain other embodiments, where a small molecule library is to be created rather than a polymer library, the anti-codon generally is associated with a reactive unit or reactant used to modify a small molecule scaffold. In certain embodiments, the reactant is linked to the anti-codon via a linker long enough to allow the reactant to come into reactive proximity with the small molecule scaffold. The linker preferably has a length and composition to permit intramolecular reactions but yet minimize intermolecular reactions. The reactants include a variety of reagents as demonstrated by the wide range of reactions that can be utilized in nucleic acid-templated synthesis and can be any chemical group, catalyst (*e*.*g*., organometallic compounds), or reactive moiety (*e*.*g*., electrophiles, nucleophiles) known in the chemical arts.

Additionally, the association between the anti-codon and the reactive unit, for example, a monomer unit or reactant, in the transfer unit may be covalent or non-covalent. The association maybe through a covalent bond and, in certain embodiments, the covalent bond may be severable.

Thus, the anti-codon can be associated with the reactant through a linker moiety. The linkage can be cleavable by light, oxidation, hydrolysis, exposure to acid, exposure to base, reduction, *etc*. Fruchtel et al. (1996) ANGEW. CHEM. INT. ED. ENGL. 35: 17 describes a variety of linkages useful in the practice of the invention. The linker facilitates contact of the reactant with the small molecule scaffold and in certain embodiments, depending on the desired reaction, positions DNA as a leaving group ("autocleavable" strategy), or may link reactive groups to the template via the "scarless" linker strategy (which yields product without leaving behind an additional atom or atoms having chemical functionality), or a "useful scar" strategy (in which a portion of the linker is left behind to be functionalized in subsequent steps following linker cleavage).

With the "autocleavable" linker strategy, the DNA-reactive group bond is cleaved as a natural consequence of the reaction. In the "scarless" linker strategy, DNA-templated reaction of one reactive group is followed by cleavage of the linker attached through a second reactive group to yield products without leaving behind additional atoms capable of providing chemical functionality. Alternatively, a "useful scar" may be utilized on the theory that it may be advantageous to introduce useful atoms and/or chemical groups as a consequence of linker cleavage. In particular, a "useful scar" is left behind following linker cleavage and can be functionalized in subsequent steps.

The anti-codon and the reactive unit (monomer unit) may also be associated through non-covalent interactions such as ionic, electrostatic, hydrogen bonding, van der Waals interactions, hydrophobic interactions, pi-stacking, *etc*. and combinations thereof To give but one example, an anti-codon may be linked to biotin, and a monomer unit linked to streptavidin. The propensity of streptavidin to bind biotin leads to the non-covalent association between the anti-codon and the monomer unit to form the transfer unit.

The specific annealing of transfer units to templates permits the use of transfer units at concentrations lower than concentrations used in many traditional organic syntheses. Thus, transfer units can be used at submillimolar concentrations (*e*.*g*. less than 100 µM, less than 10 µM, less than 1 µM, less than 100 nM, or less than 10 nM).

### III. CHEMICAL REACTIONS

A variety of compounds and/or libraries can be prepared using the methods described herein. In certain embodiments, compounds that are not, or do not resemble, nucleic acids or analogs thereof, are synthesized according to the method of the invention. In certain other embodiments, compounds that are not, or do not resemble, proteins, peptides, or analogs thereof, are synthesized according to the method of the invention.

### (i) Coupling Reactions for Small Molecule Synthesis

In some embodiments, it is possible to create compounds such as small molecules using the methods described herein. These small molecules may be like natural products, non-polymeric, and/or non-oligomeric. The substantial interest in small molecules is due in part to their use as the active ingredient in many pharmaceutical preparations although they may also be used, for example, as catalysts, materials, or additives.

In synthesizing small molecules using the method of the present invention, an evolvable template also is provided. The template can include a small molecule scaffold upon which the small molecule is to be built, or a small molecule scaffold may be added to the template. The small molecule scaffold can be any chemical compound with two or more sites for functionalization. For example, the small molecule scaffold can include a ring system (*e*.*g*., the ABCD steroid ring system found in cholesterol) with functionalizable groups coupled to the atoms making up the rings. In another example, the small molecule may be the underlying structure of a pharmaceutical agent such as morphine, epothilone or a cephalosporin antibiotic. The sites or groups to be functionalized on the small molecule scaffold may be protected using methods and protecting groups known in the art. The protecting groups used in a small molecule scaffold may be orthogonal to one another so that protecting groups can be removed one at a time.

In this embodiment, the transfer units comprise an anti-codon associated with a reactant or a building block for use in modifying, adding to, or taking away from the small molecule scaffold. The reactants or building blocks may be, for example, electrophiles (*e*.*g*., acetyl, amides, acid chlorides, esters, nitriles, imines), nucleophiles (*e*.*g*., amines, hydroxyl groups, thiols), catalysts (*e*.*g*., organometallic catalysts), or side chains. The transfer units are allowed to contact the template under hydridizing conditions. As a result of oligonucleotide annealing, the attached reactant or building block is allowed to react with a site on the small molecule scaffold. In certain embodiments, protecting groups on the small molecule template are removed one at a time from the sites to be functionalized so that the reactant of the transfer unit will react at only the desired position on the scaffold.

The reaction conditions, linker, reactant, and site to be functionalized are chosen to avoid intermolecular reactions and accelerate intramolecular reactions. Sequential or simultaneous contacting of the template with transfer units can be employed depending on the particular compound to be synthesized. In certain embodiments of special interest, the multi-step synthesis of chemical compounds is provided in which the template is contacted sequentially with two or more transfer units to facilitate multi-step synthesis of complex chemical compounds.

After the sites on the scaffold have been modified, the newly synthesized small molecule remains associated with the template that encoded its synthesis. Decoding the sequence of the template permits the deconvolution of the synthetic history and thereby the structure of the small molecule. The template can also be amplified in order to create more of the desired small molecule and/or the template can be evolved (mutagenized) to create related small molecules. The small molecule can also be cleaved from the template for purification or screening.

### (ii) Coupling Reactions for Polymer Synthesis

In certain embodiments, polymers, specifically unnatural polymers, are prepared according to the method of the present invention. The unnatural polymers that can be created using the inventive method and system include any unnatural polymers. Exemplary unnatural polymers include, but are not limited to, peptide nucleic acid (PNA) polymers, polycarbamates, polyureas, polyesters, polyacrylate, polyalkylene (*e*.*g*., polyethylene, polypropylene), polycarbonates, polypeptides with unnatural stereochemistry, polypeptides with unnatural amino acids, and combination thereof. In certain embodiments, the polymers comprise at least 10, 25, 75, 100, 125, 150 monomer units or more. The polymers synthesized using the inventive system may be used, for example, as catalysts, pharmaceuticals, metal chelators, or catalysts.

In preparing certain unnatural polymers, the monomer units attached to the anti-codons may be any monomers or oligomers capable of being joined together to form a polymer. The monomer units may be, for example, carbamates, D-amino acids, unnatural amino acids, PNAs, ureas, hydroxy acids, esters, carbonates, acrylates, or ethers. In certain embodiments, the monomer units have two reactive groups used to link the monomer unit into the growing polymer chain, as depicted in **FIG 4A****.** Preferably, the two reactive groups are not the same so that the monomer unit may be incorporated into the polymer in a directional sense, for example, at one end may be an electrophile and at the other end a nucleophile. Reactive groups may include, but are not limited to, esters, amides, carboxylic acids, activated carbonyl groups, acid chlorides, amines, hydroxyl groups, and thiols. In certain embodiments, the reactive groups are masked or protected (Greene et al. (1999) PROTECTIVE GROUPS IN ORGANIC SYNTHESIS 3rd Edition, Wiley) so that polymerization may not take place until a desired time when the reactive groups are deprotected. Once the monomer units are assembled along the nucleic acid template, initiation of the polymerization sequence results in a cascade of polymerization and deprotection steps wherein the polymerization step results in deprotection of a reactive group to be used in the subsequent polymerization step.

The monomer units to be polymerized can include two or more monomers depending on the geometry along the nucleic acid template. The monomer units to be polymerized must be able to stretch along the nucleic acid template and particularly across the distance spanned by its encoding anti-codon and optional spacer sequence. In certain embodiments, the monomer unit actually comprises two monomers, for example, a dicarbamate, a diurea, or a dipeptide. In yet other embodiments, the monomer unit comprises three or more monomers.

The monomer units may contain any chemical groups known in the art. Reactive chemical groups especially those that would interfere with polymerization, hybridization, *etc.,* are preferably masked using known protecting groups (Greene *et al.* (1999) *supra*). In general, the protecting groups used to mask these reactive groups are orthogonal to those used in protecting the groups used in the polymerization steps.

It has been discovered that, under certain circumstances, the type of chemical reaction may affect the fidelity of the polymerization process. For example, distance independent chemical reactions (for example, reactions that occur efficiently when the reactive units are spaced apart by intervening bases, for example, amine acylation reactions) may result in the spurious incorporation of the wrong monomers at a particular position of a polymer chain. In contrast, by choosing chemical reactions for template mediated syntheses that are distance dependent (for example, reactions that become inefficient the further the reactive units are spaced part via intervening bases, for example, reductive amination reactions), it is possible control the fidelity of the polymerization process.

### (iii) Functional Group Transformations

Nucleic acid-templated synthesis can be used to effect functional group transformations that either (*i*) unmask or (*ii*) interconvert functionality used in coupling reactions. By exposing or creating a reactive group within a sequence-programmed subset of a library, nucleic acid-templated functional group interconversions permit the generation of library diversity by sequential unmasking. The sequential unmasking approach offers the major advantage of enabling reactants that would normally lack the ability to be linked to a nucleic acid (for example, simple alkyl halides) to contribute to library diversity by reacting with a sequence-specified subset of templates in an intermolecular, non-templated reaction mode. This advantage significantly increases the types of structures that can be generated.

One embodiment of the invention involves deprotection or unmasking of functional groups present in a reactive unit. According to this embodiment, a nucleic acid-template is associated with a reactive unit that contains a protected functional group. A transfer unit, comprising an oligonucleotide complimentary to the template codon region and a reagent capable of removing the protecting group, is annealed to the template, and the reagent reacts with the protecting group, removing it from the reactive unit. To further functionalize the reactive unit, the exposed functional group then is subjected to a reagent not linked to a nucleic acid. In some embodiments, the reactive unit contains two or more protected functional groups. In still other embodiments, the protecting groups are orthogonal protecting groups that are sequentially removed by iterated annealing with reagents linked to transfer units.

Another embodiment of the invention involves interconversions of functional groups present on a reactive unit. According to this embodiment, a transfer unit associated with a reagent that can catalyze a reaction is annealed to a template bearing the reactive unit. A reagent not linked to a nucleic acid is added to the reaction, and the transfer unit reagent catalyzes the reaction between the unlinked reagent and the reactive unit, yielding a newly functionalized reactive unit. In some embodiments, the reactive unit contains two or more functional groups which are sequentially interconverted by iterative exposure to different transfer unit-bound reagents.

### (iv) Reaction Conditions

Nucleic acid-templated reactions can occur in aqueous or non-aqueous (*i*.*e.*, organic) solutions, or a mixture of one or more aqueous and non-aqueous solutions. In aqueous solutions, reactions can be performed at pH ranges from about 2 to about 12, or preferably from about 2 to about 10, or more preferably from about 4 to about 10. The reactions used in DNA-templated chemistry preferably should not require very basic conditions (*e*.*g*., pH > 12, pH > 10) or very acidic conditions (*e*.*g*., pH < 1, pH < 2, pH < 4), because extreme conditions may lead to degradation or modification of the nucleic acid template and/or molecule (for example, the polymer, or small molecule) being synthesized. The aqueous solution can contain one or more inorganic salts, including, but not limited to, NaCl, Na₂SO₄, KCI, Mg⁺², Mn⁺², *etc*., at various concentrations.

Organic solvents suitable for nucleic acid-templated reactions include, but are not limited to, methylene chloride, chloroform, dimethylformamide, and organic alcohols, including methanol and ethanol. To permit quantitative dissolution of reaction components in organic solvents, quaternized ammonium salts, such as, for example, long chain tetraalkylammonium salts, can be added (Jost et al. (1989) NUCLEIC ACIDS RES. 17: 2143; Mel'nikov et al. (1999) LANGMUIR 15: 1923-1928).

Nucleic acid-templated reactions may require a catalyst, such as, for example, homogeneous, heterogeneous, phase transfer, and asymmetric catalysis. In other embodiments, a catalyst is not required. The presence of additional, accessory reagents not linked to a nucleic acid are preferred in some embodiments. Useful accessory reagents can include, for example, oxidizing agents (*e*.*g*., NaIO₄); reducing agents (*e*.*g*., NaCNBH₃); activating reagents (*e*.*g*., EDC, NHS, and sulfo-NHS); transition metals such as nickel (*e*.*g*., Ni(NO₃)₂), rhodium (*e*.*g*. RhCl₃), ruthenium (*e*.*g*. RuCl₃), copper (*e*.*g*. Cu(NO₃)₂), cobalt (*e*.*g*. CoCl₂), iron (*e*.*g*. Fe(NO₃)₃), osmium (*e*.*g*. OsO₄), titanium (*e*.*g*. TiCl₄ or titanium tetraisopropoxide), palladium (*e*.*g*. NaPdCl₄), or Ln; transition metal ligands (*e*.*g*., phosphines, amines, and halides); Lewis acids; and Lewis bases.

Reaction conditions preferably are optimized to suit the nature of the reactive units and oligonucleotides used.

### (v) Classes of Chemical Reactions

Known chemical reactions for synthesizing polymers, small molecules, or other molecules can be used in nucleic acid-templated reactions. Thus, reactions such as those listed in *March's Advanced Organic Chemistry, Organic Reactions, Organic Syntheses*, organic text books, journals such as *Journal of the American Chemical Society, Journal of Organic Chemistry, Tetrahedron, etc*., and Carruther's *Some Modern Methods of Organic Chemistry* can be used. The chosen reactions preferably are compatible with nucleic acids such as DNA or RNA or are compatible with the modified nucleic acids used as the template.

Reactions useful in nucleic-acid templated chemistry include, for example, substitution reactions, carbon-carbon bond forming reactions, elimination reactions, acylation reactions, and addition reactions. An illustrative but not exhaustive list of aliphatic nucleophilic substitution reactions useful in the present invention includes, for example, S_{N}2 reactions, S_{N}1 reactions, S_{N}i reactions, allylic rearrangements, nucleophilic substitution at an aliphatic trigonal carbon, and nucleophilic substation at a vinylic carbon.

Specific aliphatic nucleophilic substitution reactions with oxygen nucleophiles include, for example, hydrolysis of alkyl halides, hydrolysis of gen-dihalides, hydrolysis of 1,1,1-trihalides, hydrolysis of alkyl esters or inorganic acids, hydrolysis of diazo ketones, hydrolysis of acetal and enol ethers, hydrolysis of epoxides, hydrolysis of acyl halides, hydrolysis of anhydrides, hydrolysis of carboxylic esters, hydrolysis of amides, alkylation with alkyl halides (Williamson Reaction), epoxide formation, alkylation with inorganic esters, alkylation with diazo compounds, dehydration of alcohols, transetherification, alcoholysis of epoxides, alkylation with onium salts, hydroxylation of silanes, alcoholysis of acyl halides, alcoholysis of anhydrides, esterfication of carboxylic acids, alcoholysis of carboxylic esters (transesterfication), alcoholysis of amides, alkylation of carboxylic acid salts, cleavage of ether with acetic anhydride, alkylation of carboxylic acids with diazo compounds, acylation of caroxylic acids with acyl halides, acylation of carboxylic acids with carboxylic acids, formation of oxonium salts, preparation of peroxides and hydroperoxides, preparation of inorganic esters (*e*.*g*., nitrites, nitrates, sulfonates), preparation of alcohols from amines, and preparation of mixed organic-inorganic anhydrides.

Specific aliphatic nucleophilic substitution reactions with sulfur nucleophiles, which tend to be better nucleophiles than their oxygen analogs, include, for example, attack by SH at an alkyl carbon to form thiols, attack by S at an alkyl carbon to form thioethers, attack by SH or SR at an acyl carbon, formation of disulfides, formation of Bunte salts, alkylation of sulfinic acid salts, and formation of alkyl thiocyanates.

Aliphatic nucleophilic substitution reactions with nitrogen nucleophiles include, for example, alkylation of amines, *N*-arylation of amines, replacement of a hydroxy by an amino group, transamination, transamidation, alkylation of amines with diazo compounds, amination of epoxides, amination of oxetanes, amination of aziridines, amination of alkanes, formation of isocyanides, acylation of amines by acyl halides, acylation of amines by anhydrides, acylation of amines by carboxylic acids, acylation of amines by carboxylic esters, acylation of amines by amides, acylation of amines by other acid derivatives, *N*-alkylation or *N*-arylation of amides and imides, *N*-acylation of amides and imides, formation of aziridines from epoxides, formation of nitro compounds, formation of azides, formation of isocyanates and isothiocyanates, and formation of azoxy compounds.

Aliphatic nucleophilic substitution reactions with halogen nucleophiles include, for example, attack at an alkyl carbon, halide exchange, formation of alkyl halides from esters of sulfuric and sulfonic acids, formation of alkyl halides from alcohols, formation of alkyl halides from ethers, formation of halohydrins from epoxides, cleavage of carboxylic esters with lithium iodide, conversion of diazo ketones to α-halo ketones, conversion of amines to halides, conversion of tertiary amines to cyanamides (the von Braun reaction), formation of acyl halides from carboxylic acids, and formation of acyl halides from acid derivatives.

Aliphatic nucleophilic substitution reactions using hydrogen as a nucleophile include, for example, reduction of alkyl halides, reduction of tosylates, other sulfonates, and similar compounds, hydrogenolysis of alcohols, hydrogenolysis of esters (Barton-McCombie reaction), hydrogenolysis of nitriles, replacement of alkoxyl by hydrogen, reduction of epoxides, reductive cleavage of carboxylic esters, reduction of a C-N bond, desulfurization, reduction of acyl halides, reduction of carboxylic acids, esters, and anhydrides to aldehydes, and reduction of amides to aldehydes.

Although certain carbon nucleophiles may be too nucleophilic and/or basic to be used in certain embodiments of the invention, aliphatic nucleophilic substitution reactions using carbon nucleophiles include, for example, coupling with silanes, coupling of alkyl halides (the Wurtz reaction), the reaction of alkyl halides and sulfonate esters with Group I (I A) and II (II A) organometallic reagents, reaction of alkyl halides and sulfonate esters with organocuprates, reaction of alkyl halides and sulfonate esters with other organometallic reagents, allylic and propargylic coupling with a halide substrate, coupling of organometallic reagents with esters of sulfuric and sulfonic acids, sulfoxides, and sulfones, coupling involving alcohols, coupling of organometallic reagents with carboxylic esters, coupling of organometallic reagents with compounds containing an esther linkage, reaction of organometallic reagents with epoxides, reaction of organometallics with aziridine, alkylation at a carbon bearing an active hydrogen, alkylation of ketones, nitriles, and carboxylic esters, alkylation of carboxylic acid salts, alkylation at a position a to a heteroatom (alkylation of 1,3-dithianes), alkylation of dihydro-1,3-oxazine (the Meyers synthesis of aldehydes, ketones, and carboxylic acids), alkylation with trialkylboranes, alkylation at an alkynyl carbon, preparation of nitriles, direct conversion of alkyl halides to aldehydes and ketones, conversion of alkyl halides, alcohols, or alkanes to carboxylic acids and their derivatives, the conversion of acyl halides to ketones with organometallic compounds, the conversion of anhydrides, carboxylic esters, or amides to ketones with organometallic compounds, the coupling of acyl halides, acylation at a carbon bearing an active hydrogen, acylation of carboxylic esters by carboxylic esters (the Claisen and Dieckmann condensation), acylation of ketones and nitriles with carboxylic esters, acylation of carboxylic acid salts, preparation of acyl cyanides, and preparation of diazo ketones, ketonic decarboxylation.

Reactions which involve nucleophilic attack at a sulfonyl sulfur atom may also be used in the present invention and include, for example, hydrolysis of sulfonic acid derivatives (attack by OH), formation of sulfonic esters (attack by OR), formation of sulfonamides (attack by nitrogen), formation of sulfonyl halides (attack by halides), reduction of sulfonyl chlorides (attack by hydrogen), and preparation of sulfones (attack by carbon).

Aromatic electrophilic substitution reactions may also be used in nucleotide-templated chemistry. Hydrogen exchange reactions are examples of aromatic electrophilic substitution reactions that use hydrogen as the electrophile. Aromatic electrophilic substitution reactions which use nitrogen electrophiles include, for example, nitration and nitro-de-hydrogenation, nitrosation of nitroso-de-hydrogenation, diazonium coupling, direct introduction of the diazonium group, and amination or amino-de-hydrogenation. Reactions of this type with sulfur electrophiles include, for example, sulfonation, sulfo-de-hydrogenation, halosulfonation, halosulfo-de-hydrogenation, sulfurization, and sulfonylation. Reactions using halogen electrophiles include, for example, halogenation, and halo-de-hydrogenation. Aromatic electrophilic substitution reactions with carbon electrophiles include, for example, Friedel-Crafts alkylation, alkylation, alkyl-de-hydrogenation, Friedel-Crafts arylation (the Scholl reaction), Friedel-Crafts acylation, formylation with disubstituted formamides, formylation with zinc cyanide and HCl (the Gatterman reaction), formylation with chloroform (the Reimer-Tiemann reaction), other formylations, formyl-de-hydrogenation, carboxylation with carbonyl halides, carboxylation with carbon dioxide (the Kolbe-Schmitt reaction), amidation with isocyanates, *N-*alkylcarbamoyl-de-hydrogenation, hydroxyalkylation, hydroxyalkyl-de-hydrogenation, cyclodehydration of aldehydes and ketones, haloalkylation, halo-de-hydrogenation, aminoalkylation, amidoalkylation, dialkylaminoalkylation, dialkylamino-de-hydrogenation, thioalkylation, acylation with nitriles (the Hoesch reaction), cyanation, and cyano-dehydrogenation. Reactions using oxygen electrophiles include, for example, hydroxylation and hydroxy-de-hydrogenation.

Rearrangement reactions include, for example, the Fries rearrangement, migration of a nitro group, migration of a nitroso group (the Fischer-Hepp Rearrangement), migration of an arylazo group, migration of a halogen (the Orton rearrangement), migration of an alkyl group, *etc.* Other reaction on an aromatic ring include the reversal of a Friedel-Crafts alkylation, decarboxylation of aromatic aldehydes, decarboxylation of aromatic acids, the Jacobsen reaction, deoxygenation, desulfonation, hydro-de-sulfonation, dehalogenation, hydro-de-halogenation, and hydrolysis of organometallic compounds.

Aliphatic electrophilic substitution reactions are also useful. Reactions using the S_{E}1, S_{E}2 (front), S_{E}2 (back), S_{E}i, addition-elimination, and cyclic mechanisms can be used in the present invention. Reactions of this type with hydrogen as the leaving group include, for example, hydrogen exchange (deuterio-de-hydrogenation, deuteriation), migration of a double bond, and keto-enol tautomerization. Reactions with halogen electrophiles include, for example, halogenation of aldehydes and ketones, halogenation of carboxylic acids and acyl halides, and halogenation of sulfoxides and sulfones. Reactions with nitrogen electrophiles include, for example, aliphatic diazonium coupling, nitrosation at a carbon bearing an active hydrogen, direct formation of diazo compounds, conversion of amides to α-azido amides, direct amination at an activated position, and insertion by nitrenes. Reactions with sulfur or selenium electrophiles include, for example, sulfenylation, sulfonation, and selenylation of ketones and carboxylic esters. Reactions with carbon electrophiles include, for example, acylation at an aliphatic carbon, conversion of aldehydes to β-keto esters or ketones, cyanation, cyano-de-hydrogenation, alkylation of alkanes, the Stork enamine reaction, and insertion by carbenes. Reactions with metal electrophiles include, for example, metalation with organometallic compounds, metalation with metals and strong bases, and conversion of enolates to silyl enol ethers. Aliphatic electrophilic substitution reactions with metals as leaving groups include, for example, replacement of metals by hydrogen, reactions between organometallic reagents and oxygen, reactions between organometallic reagents and peroxides, oxidation of trialkylboranes to borates, conversion of Grignard reagents to sulfur compounds, halo-de-metalation, the conversion of organometallic compounds to amines, the conversion of organometallic compounds to ketones, aldehydes, carboxylic esters and amides, cyano-de-metalation, transmetalation with a metal, transmetalation with a metal halide, transmetalation with an organometallic compound, reduction of alkyl halides, metallo-de-halogenation, replacement of a halogen by a metal from an organometallic compound, decarboxylation of aliphatic acids, cleavage of alkoxides, replacement of a carboxyl group by an acyl group, basic cleavage of β-keto esters and β-diketones, haloform reaction, cleavage of non-enolizable ketones, the Haller-Bauer reaction, cleavage of alkanes, decyanation, and hydro-de-cyanation. Electrophlic substitution reactions at nitrogen include, for example, diazotization, conversion of hydrazines to azides, *N*-nitrosation, *N*-nitroso-de-hydrogenation, conversion of amines to azo compounds, *N*-halogenation, *N*-halo-de-hydrogenation, reactions of amines with carbon monoxide, and reactions of amines with carbon dioxide.

Aromatic nucleophilic substitution reactions may also be used in the present invention. Reactions proceeding via the S_{N}Ar mechanism, the S_{N}1 mechanism, the benzyne mechanism, the S_{RN}1 mechanism, or other mechanism, for example, can be used. Aromatic nucleophilic substitution reactions with oxygen nucleophiles include, for example, hydroxy-de-halogenation, alkali fusion of sulfonate salts, and replacement of OR or OAr. Reactions with sulfur nucleophiles include, for example, replacement by SH or SR. Reactions using nitrogen nucleophiles include, for example, replacement by NH₂, NHR, or NR₂, and replacement of a hydroxy group by an amino group. Reactions with halogen nucleophiles include, for example, the introduction halogens. Aromatic nucleophilic substitution reactions with hydrogen as the nucleophile include, for example, reduction of phenols and phenolic esters and ethers, and reduction of halides and nitro compounds. Reactions with carbon nucleophiles include, for example, the Rosenmund-von Braun reaction, coupling of organometallic compounds with aryl halides, ethers, and carboxylic esters, arylation at a carbon containing an active hydrogen, conversions of aryl substrates to carboxylic acids, their derivatives, aldehydes, and ketones, and the Ullmann reaction. Reactions with hydrogen as the leaving group include, for example, alkylation, arylation, and amination of nitrogen heterocycles. Reactions with N₂⁺ as the leaving group include, for example, hydroxy-de-diazoniation, replacement by sulfur-containing groups, iodo-de-diazoniation, and the Schiemann reaction. Rearrangement reactions include, for example, the von Richter rearrangement, the Sommelet-Hauser rearrangement, rearrangement of aryl hydroxylamines, and the Smiles rearrangement.

Reactions involving free radicals can also be used, although the free radical reactions used in nucleotide-templated chemistry should be carefully chosen to avoid modification or cleavage of the nucleotide template. With that limitation, free radical substitution reactions can be used in the present invention. Particular free radical substitution reactions include, for example, substitution by halogen, halogenation at an alkyl carbon, allylic halogenation, benzylic halogenation, halogenation of aldehydes, hydroxylation at an aliphatic carbon, hydroxylation at an aromatic carbon, oxidation of aldehydes to carboxylic acids, formation of cyclic ethers, formation of hydroperoxides, formation of peroxides, acyloxylation, acyloxy-de-hydrogenation, chlorosulfonation, nitration of alkanes, direct conversion of aldehydes to amides, amidation and amination at an alkyl carbon, simple coupling at a susceptible position, coupling of alkynes, arylation of aromatic compounds by diazonium salts, arylation of activated alkenes by diazonium salts (the Meerwein arylation), arylation and alkylation of alkenes by organopalladium compounds (the Heck reaction), arylation and alkylation of alkenes by vinyltin compounds (the Stille reaction), alkylation and arylation of aromatic compounds by peroxides, photochemical arylation of aromatic compounds, alkylation, acylation, and carbalkoxylation of nitrogen heterocycles Particular reactions in which N₂⁺ is the leaving group include, for example, replacement of the diazonium group by hydrogen, replacement of the diazonium group by chlorine or bromine, nitro-de-diazoniation, replacement of the diazonium group by sulfur-containing groups, aryl dimerization with diazonium salts, methylation of diazonium salts, vinylation of diazonium salts, arylation of diazonium salts, and conversion of diazonium salts to aldehydes, ketones, or carboxylic acids. Free radical substitution reactions with metals as leaving groups include, for example, coupling of Grignard reagents, coupling of boranes, and coupling of other organometallic reagents. Reaction with halogen as the leaving group are included. Other free radical substitution reactions with various leaving groups include, for example, desulfurization with Raney Nickel, conversion of sulfides to organolithium compounds, decarboxylative dimerization (the Kolbe reaction), the Hunsdiecker reaction, decarboxylative allylation, and decarbonylation of aldehydes and acyl halides.

Reactions involving additions to carbon-carbon multiple bonds are also used in nucleotide-templated chemistry. Any mechanism may be used in the addition reaction including, for example, electrophilic addition, nucleophilic addition, free radical addition, and cyclic mechanisms. Reactions involving additions to conjugated systems can also be used. Addition to cyclopropane rings can also be utilized. Particular reactions include, for example, isomerization, addition of hydrogen halides, hydration of double bonds, hydration of triple bonds, addition of alcohols, addition of carboxylic acids, addition of H₂S and thiols, addition of ammonia and amines, addition of amides, addition of hydrazoic acid, hydrogenation of double and triple bonds, other reduction of double and triple bonds, reduction of the double and triple bonds of conjugated systems, hydrogenation of aromatic rings, reductive cleavage of cyclopropanes, hydroboration, other hydrometalations, addition of alkanes, addition of alkenes and/or alkynes to alkenes and/or alkynes (*e*.*g*., pi-cation cyclization reactions, hydro-alkenyl-addition), ene reactions, the Michael reaction, addition of organometallics to double and triple bonds not conjugated to carbonyls, the addition of two alkyl groups to an alkyne, 1,4-addition of organometallic compounds to activated double bonds, addition of boranes to activated double bonds, addition of tin and mercury hydrides to activated double bonds, acylation of activated double bonds and of triple bonds, addition of alcohols, amines, carboxylic esters, aldehydes, *etc.,* carbonylation of double and triple bonds, hydrocarboxylation, hydroformylation, addition of aldehydes, addition of HCN, addition of silanes, radical addition, radical cyclization, halogenation of double and triple bonds (addition of halogen, halogen), halolactonization, halolactamization, addition of hypohalous acids and hypohalites (addition of halogen, oxygen), addition of sulfur compounds (addition of halogen, sulfur), addition of halogen and an amino group (addition of halogen, nitrogen), addition of NOX and NO₂X (addition of halogen, nitrogen), addition of XN₃ (addition of halogen, nitrogen), addition of alkyl halides (addition of halogen, carbon), addition of acyl halides (addition of halogen, carbon), hydroxylation (addition of oxygen, oxygen) (*e*.*g*., asymmetric dihydroxylation reaction with OsO₄), dihydroxylation of aromatic rings, epoxidation (addition of oxygen, oxygen) (*e*.*g*., Sharpless asymmetric epoxidation), photooxidation of dienes (addition of oxygen, oxygen), hydroxysulfenylation (addition of oxygen, sulfur), oxyamination (addition of oxygen, nitrogen), diamination (addition of nitrogen, nitrogen), formation of aziridines (addition of nitrogen), aminosulfenylation (addition of nitrogen, sulfur), acylacyloxylation and acylamidation (addition of oxygen, carbon or nitrogen, carbon), 1,3-dipolar addition (addition of oxygen, nitrogen, carbon), Diels-Alder reaction, heteroatom Diels-Alder reaction, all carbon 3 +2 cycloadditions, dimerization of alkenes, the addition of carbenes and carbenoids to double and triple bonds, trimerization and tetramerization of alkynes, and other cycloaddition reactions.

In addition to reactions involving additions to carbon-carbon multiple bonds, addition reactions to carbon-hetero multiple bonds can be used in nucleotide-templated chemistry. Exemplary reactions include, for example, the addition of water to aldehydes and ketones (formation of hydrates), hydrolysis of carbon-nitrogen double bond, hydrolysis of aliphatic nitro compounds, hydrolysis of nitriles, addition of alcohols and thiols to aldehydes and ketones, reductive alkylation of alcohols, addition of alcohols to isocyanates, alcoholysis of nitriles, formation of xanthates, addition of H₂S and thiols to carbonyl compounds, formation of bisulfite addition products, addition of amines to aldehydes and ketones, addition of amides to aldehydes, reductive alkylation of ammonia or amines, the Mannich reaction, the addition of amines to isocyanates, addition of ammonia or amines to nitriles, addition of amines to carbon disulfide and carbon dioxide, addition of hydrazine derivative to carbonyl compounds, formation of oximes, conversion of aldehydes to nitriles, formation of gem-dihalides from aldehydes and ketones, reduction of aldehydes and ketones to alcohols, reduction of the carbon-nitrogen double bond, reduction of nitriles to amines, reduction of nitriles to aldehydes, addition of Grignard reagents and organolithium reagents to aldehydes and ketones, addition of other organometallics to aldehydes and ketones, addition of trialkylallylsilanes to aldehydes and ketones, addition of conjugated alkenes to aldehydes (the Baylis-Hillman reaction), the Reformatsky reaction, the conversion of carboxylic acid salts to ketones with organometallic compounds, the addition of Grignard reagents to acid derivatives, the addition of organometallic compounds to CO₂ and CS₂, addition of organometallic compounds to C=N compounds, addition of carbenes and diazoalkanes to C=N compounds, addition of Grignard reagents to nitriles and isocyanates, the Aldol reaction, Mukaiyama Aldol and related reactions, Aldol-type reactions between carboxylic esters or amides and aldehydes or ketones, the Knoevenagel reaction (*e*.*g*., the Nef reaction, the Favorskii reaction), the Peterson alkenylation reaction, the addition of active hydrogen compounds to CO₂ and CS₂, the Perkin reaction, Darzens glycidic ester condensation, the Tollens' reaction, the Wittig reaction, the Tebbe alkenylation, the Petasis alkenylation, alternative alkenylations, the Thorpe reaction, the Thorpe-Ziegler reaction, addition of silanes, formation of cyanohydrins, addition of HCN to C=N and C=N bonds, the Prins reaction, the benzoin condensation, addition of radicals to C=O, C=S, C=N compounds, the Ritter reaction, acylation of aldehydes and ketones, addition of aldehydes to aldehydes, the addition of isocyanates to isocyanates (formation of carbodiimides), the conversion of carboxylic acid salts to nitriles, the formation of epoxides from aldehydes and ketones, the formation of episulfides and episulfones, the formation of β-lactones and oxetanes (*e*.*g*., the Paterno-Büchi reaction), the formation of β-lactams, *etc*. Reactions involving addition to isocyanides include the addition of water to isocyanides, the Passerini reaction, the Ug reaction, and the formation of metalated aldimines.

Elimination reactions, including α, β, and γ eliminations, as well as extrusion reactions, can be performed using nucleotide-templated chemistry, although the strength of the reagents and conditions employed should be considered. Preferred elimination reactions include reactions that go by E1, E2, E1cB, or E2C mechanisms. Exemplary reactions include, for example, reactions in which hydrogen is removed from one side (*e*.*g*., dehydration of alcohols, cleavage of ethers to alkenes, the Chugaev reaction, ester decomposition, cleavage of quarternary ammonium hydroxides, cleavage of quaternary ammonium salts with strong bases, cleavage of amine oxides, pyrolysis ofketo-ylids, decomposition of toluene-p-solfonylhydrazones, cleavage of sulfoxides, cleavage of selenoxides, cleavage of sulfornes, dehydrogalogenation of alkyl halides, dehydrohalogenation of acyl halides, dehydrohalogenation of sulfonyl halides, elimination of boranes, conversion of alkenes to alkynes, decarbonylation of acyl halides), reactions in which neither leaving atom is hydrogen (*e*.*g*., deoxygenation of vicinal diols, cleavage of cyclic thionocarbonates, conversion of epoxides to episulfides and alkenes, the Ramberg-Bäcklund reaction, conversion of aziridines to alkenes, dehalogenation of vicinal dihalides, dehalogenation of α-halo acyl halides, and elimination of a halogen and a hetero group), fragmentation reactions (*i*.*e*., reactions in which carbon is the positive leaving group or the electrofuge, such as, for example, fragmentation of γ-amino and γ-hydroxy halides, fragmentation of 1,3-diols, decarboxylation of β-hydroxy carboxylic acids, decarboxylation of β-lactones, fragmentation of α,β-epoxy hydrazones, elimination of CO from briged bicyclic compounds, and elimination of CO₂ from bridged bicyclic compounds), reactions in which C≡N or C=N bonds are formed (*e*.*g*., dehydration of aldoximes or similar compounds, conversion of ketoximes to nitriles, dehydration of unsubstituted amides, and conversion of N-alkylformamides to isocyanides), reactions in which C=O bonds are formed (*e*.*g*., pyrolysis of β-hydroxy alkenes), and reactions in which N=N bonds are formed (*e*.*g*., eliminations to give diazoalkenes). Extrusion reactions include, for example, extrusion of N₂ from pyrazolines, extrusion of N₂ from pyrazoles, extrusion of N₂ from triazolines, extrusion of CO, extrusion of CO₂, extrusion of SO₂, the Story synthesis, and alkene synthesis by twofold extrusion.

Rearrangements, including, for example, nucleophilic rearrangements, electrophilic rearrangements, prototropic rearrangements, and free-radical rearrangements, can also be performed using nucleotide-templated chemistry. Both 1,2 rearrangements and non-1,2 rearrangements can be performed. Exemplary reactions include, for example, carbon-to-carbon migrations of R, H, and Ar (*e*.*g*., Wagner-Meerwein and related reactions, the Pinacol rearrangement, ring expansion reactions, ring contraction reactions, acid-catalyzed rearrangements of aldehydes and ketones, the dienone-phenol rearrangement, the Favorskii rearrangement, the Arndt-Eistert synthesis, homologation of aldehydes, and homologation of ketones), carbon-to-carbon migrations of other groups (*e*.*g*., migrations of halogen, hydroxyl, amino, *etc*.; migration of boron; and the Neber rearrangement), carbon-to-nitrogen migrations of R and Ar (*e*.*g*., the Hofmann rearrangement, the Curtius rearrangement, the Lossen rearrangement, the Schmidt reaction, the Beckman rearrangement, the Stieglits rearrangement, and related rearrangements), carbon-to-oxygen migrations of R and Ar (*e*.*g*., the Baeyer-Villiger rearrangement and rearrangment of hydroperoxides), nitrogen-to-carbon, oxygen-to-carbon, and sulfur-to-carbon migration (*e*.*g*., the Stevens rearrangement, and the Wittig rearrangement), boron-to-carbon migrations (*e*.*g*., conversion of boranes to alcohols (primary or otherwise), conversion of boranes to aldehydes, conversion of boranes to carboxylic acids, conversion of vinylic boranes to alkenes, formation of alkynes from boranes and acetylides, formation of alkenes from boranes and acetylides, and formation of ketones from boranes and acetylides), electrocyclic rearrangements (*e*.*g*., of cyclobutenes and 1,3-cyclohexadienes, or conversion of stilbenes to phenanthrenes), sigmatropic rearrangements (*e*.*g*., (1,j) sigmatropic migrations of hydrogen, (1,j) sigmatropic migrations of carbon, conversion of vinylcyclopropanes to cyclopentenes, the Cope rearrangement, the Claisen rearrangement, the Fischer indole synthesis, (2,3) sigmatropic rearrangements, and the benzidine rearrangement), other cyclic rearrangements (*e*.*g*., metathesis of alkenes, the di-π-methane and related rearrangements, and the Hofmann-Löffler and related reactions), and non-cyclic rearrangements (*e*.*g*., hydride shifts, the Chapman rearrangement, the Wallach rearrangement, and dyotropic rearrangements).

Oxidative and reductive reactions may also be performed using nucleotide-templated chemistry. Exemplary reactions may involve, for example, direct electron transfer, hydride transfer, hydrogen-atom transfer, formation of ester intermediates, displacement mechanisms, or addition-elimination mechanisms. Exemplary oxidations include, for example, eliminations of hydrogen (*e*.*g*., aromatization of six-membered rings, dehydrogenations yielding carbon-carbon double bonds, oxidation or dehydrogenation of alcohols to aldehydes and ketones, oxidation of phenols and aromatic amines to quinones, oxidative cleavage of ketones, oxidative cleavage of aldehydes, oxidative cleavage of alcohols, ozonolysis, oxidative cleavage of double bonds and aromatic rings, oxidation of aromatic side chains, oxidative decarboxylation, and bisdecarboxylation), reactions involving replacement of hydrogen by oxygen (*e*.*g*., oxidation of methylene to carbonyl, oxidation of methylene to OH, CO₂R, or OR, oxidation of arylmethanes, oxidation of ethers to carboxylic esters and related reactions, oxidation of aromatic hydrocarbons to quinones, oxidation of amines or nitro compounds to aldehydes, ketones, or dihalides, oxidation of primary alcohols to carboxylic acids or carboxylic esters, oxidation of alkenes to aldehydes or ketones, oxidation of amines to nitroso compounds and hydroxylamines, oxidation of primary amines, oximes, azides, isocyanates, or notroso compounds, to nitro compounds, oxidation ofthiols and other sulfur compounds to sulfonic acids), reactions in which oxygen is added to the subtrate (*e*.*g*., oxidation of alkynes to α-diketones, oxidation of tertiary amines to amine oxides, oxidation of thioesters to sulfoxides and sulfones, and oxidation of carboxylic acids to peroxy acids), and oxidative coupling reactions (*e*.*g*., coupling involving carbanoins, dimerization of silyl enol ethers or of lithium enolates, and oxidation of thiols to disulfides).

Exemplary reductive reactions include, for example, reactions involving replacement of oxygen by hydrogen (*e*.*g*., reduction of carbonyl to methylene in aldehydes and ketones, reduction of carboxylic acids to alcohols, reduction of amides to amines, reduction of carboxylic esters to ethers, reduction of cyclic anhydrides to lactones and acid derivatives to alcohols, reduction of carboxylic esters to alcohols, reduction of carboxylic acids and esters to alkanes, complete reduction of epoxides, reduction of nitro compounds to amines, reduction of nitro compounds to hydroxylamines, reduction of nitroso compounds and hydroxylamines to amines, reduction of oximes to primary amines or aziridines, reduction of azides to primary amines, reduction of nitrogen compounds, and reduction of sulfonyl halides and sulfonic acids to thiols), removal of oxygen from the substrate (*e*.*g*., reduction of amine oxides and azoxy compounds, reduction of sulfoxides and sulfones, reduction of hydroperoxides and peroxides, and reduction of aliphatic nitro compounds to oximes or nitriles), reductions that include cleavage (*e*.*g*., dealkylation of amines and amides, reduction of azo, azoxy, and hydrazo compounds to amines, and reduction of disulfides to thiols), reductive couplic reactions (*e*.*g*., bimolecular reduction of aldehydes and ketones to 1,2-diols, bimolecular reduction of aldehydes or ketones to alkenes, acyloin ester condensation, reduction of nitro to azoxy compounds, and reduction of nitro to azo compounds), and reductions in which an organic substrate is both oxidized and reduced (*e*.*g*., the Cannizzaro reaction, the Tishchenko reaction, the Pummerer rearrangement, and the Willgerodt reaction).

### IV. SELECTION AND SCREENING

Selection and/or screening for reaction products with desired activities (such as catalytic activity, binding affinity, or a particular effect in an activity assay) may be performed using methodologies known and used in the art. For example, affinity selections may be performed according to the principles used in library-based selection methods such as phage display, polysome display, and mRNA-fusion protein displayed peptides. Selection for catalytic activity may be performed by affinity selections on transition-state analog affinity columns (Baca et al. (1997) PROC. NATL. ACAD. SCI. USA 94(19): 10063-8) or by function-based selection schemes (Pedersen et al. (1998) PROC. NATL. ACAD. SCI. USA 95(18): 10523-8). Since minute quantities of DNA (∼10⁻²⁰ mol) can be amplified by PCR (Kramer et al. (1999) CURRENT PROTOCOLS IN MOLECULAR BIOLOGY (ed. Ausubel, F. M.) 15.1-15.3, Wiley), these selections can be conducted on a scale ten or more orders of magnitude less than that required for reaction analysis by current methods, making a truly broad search both economical and efficient.

### (i) Selection for Binding to Target Molecule

The templates and reaction products can be selected (or screened) for binding to a target molecule. In this context, selection or partitioning means any process whereby a library member bound to a target molecule is separated from library members not bound to target molecules. Selection can be accomplished by various methods known in the art.

The templates of the present invention contain a built-in function for direct selection and amplification. In most applications, binding to a target molecule preferably is selective, such that the template and the resulting reaction product bind preferentially with a specific target molecule, perhaps preventing or inducing a specific biological effect. Ultimately, a binding molecule identified using the present invention may be useful as a therapeutic and/or diagnostic agent. Once the selection is complete, the selected templates optionally can be amplified and sequenced. The selected reaction products, if present in sufficient quantity, can be separated from the templates, purified (*e*.*g*., by HPLC, column chromatography, or other chromatographic method), and further characterized.

### (ii) Target Molecules

Binding assays provide a rapid means for isolating and identifying reaction products that bind to, for example, a surface (such as metal, plastic, composite, glass, ceramics, rubber, skin, or tissue); a polymer; a catalyst; or a target biomolecule such as a nucleic acid, a protein (including enzymes, receptors, antibodies, and glycoproteins), a signal molecule (such as cAMP, inositol triphosphate, peptides, or prostaglandins), a carbohydrate, or a lipid. Binding assays can be advantageously combined with activity assays for the effect of a reaction product on a function of a target molecule.

The selection strategy can be carried out to allow selection against almost any target. Importantly, the selection strategy does not require any detailed structural information about the target molecule or about the molecules in the libraries. The entire process is driven by the binding affinity involved in the specific recognition and binding of the molecules in the library to a given target. Examples of various selection procedures are described below.

The libraries of the present invention can contain molecules that could potentially bind to any known or unknown target. The binding region of a target molecule could include a catalytic site of an enzyme, a binding pocket on a receptor (for example, a G-protein coupled receptor), a protein surface area involved in a protein-protein or protein-nucleic acid interaction (preferably a hot-spot region), or a specific site on DNA (such as the major groove). The natural function of the target could be stimulated (agonized), reduced (antagonized), unaffected, or completely changed by the binding of the reaction product. This will depend on the precise binding mode and the particular binding site the reaction product occupies on the target.

Functional sites (such as protein-protein interaction or catalytic sites) on proteins often are more prone to bind molecules than are other more neutral surface areas on a protein. In addition, these functional sites normally contain a smaller region that seems to be primarily responsible for the binding energy: the so-called "hot-spot regions" (Wells, et al. (1993) RECENT PROG. HORMONE RES. 48: 253- 262). This phenomenon facilitates selection for molecules affecting the biological function of a certain target.

The linkage between the template molecule and reaction product allows rapid identification of binding molecules using various selection strategies. This invention broadly permits identifying binding molecules for any known target molecule. In addition, novel unknown targets can be discovered by isolating binding molecules against unknown antigens (epitopes) and using these binding molecules for identification and validation. In another preferred embodiment, the target molecule is designed to mimic a transition state of a chemical reaction; one or more reaction products resulting from the selection may stabilize the transition state and catalyze the chemical reaction.

### (iii) Binding Assays

The template-directed synthesis of the invention permits selection procedures analogous to other display methods such as phage display (Smith (1985) SCIENCE 228: 1315-1317). Phage display selection has been used successfully on peptides (Wells et al. (1992) CURR. OP. STRUCT. BIOL. 2: 597-604), proteins (Marks et al. (1992) J. BIOL. CHEM. 267: 16007-16010) and antibodies (Winter et al. (1994) ANNU. REV. IMMUNOL. 12: 433-455). Similar selection procedures also are exploited for other types of display systems such as ribosome display Mattheakis et al. (1994) PROC. NATL. ACAD. SCI. 91: 9022-9026) and mRNA display (Roberts, et al. (1997) PROC. NATL. ACAD. SCI. 94:12297-302). The libraries of the present invention, however, allow direct selection of target-specific molecules without requiring traditional ribosome-mediated translation. The present invention also allows the display of small molecules which have not previously been synthesized directly from a nucleic acid template.

Selection of binding molecules from a library can be performed in any format to identify optimal binding molecules. Binding selections typically involve immobilizing the desired target molecule, adding a library of potential binders, and removing non-binders by washing. When the molecules showing low affinity for an immobilized target are washed away, the molecules with a stronger affinity generally remain attached to the target. The enriched population remaining bound to the target after stringent washing is preferably eluted with, for example, acid, chaotropic salts, heat, competitive elution with a known ligand or by proteolytic release of the target and/or of template molecules. The eluted templates are suitable for PCR, leading to many orders of amplification, whereby essentially each selected template becomes available at a greatly increased copy number for cloning, sequencing, and/or further enrichment or diversification.

In a binding assay, when the concentration of ligand is much less than that of the target (as it would be during the selection of a DNA-templated library), the fraction of ligand bound to target is determined by the effective concentration of the target protein. The fraction of ligand bound to target is a sigmoidal function of the concentration of target, with the midpoint (50% bound) at [target] = *K*_{d} of the ligand-target complex. This relationship indicates that the stringency of a specific selection - the minimum ligand affinity required to remain bound to the target during the selection - is determined by the target concentration. Therefore, selection stringency is controllable by varying the effective concentration of target.

The target molecule (peptide, protein, DNA or other antigen) can be immobilized on a solid support, for example, a container wall, a wall of a microtiter plate well. The library preferably is dissolved in aqueous binding buffer in one pot and equilibrated in the presence of immobilized target molecule. Non-binders are washed away with buffer. Those molecules that may be binding to the target molecule through their attached DNA templates rather than through their synthetic moieties can be eliminated by washing the bound library with unfunctionalized templates lacking PCR primer binding sites. Remaining bound library members then can be eluted, for example, by denaturation.

Alternatively, the target molecule can be immobilized on beads, particularly if there is doubt that the target molecule will adsorb sufficiently to a container wall, as may be the case for an unfolded target eluted from an SDS-PAGE gel. The derivatized beads can then be used to separate high-affinity library members from nonbinders by simply sedimenting the beads in a benchtop centrifuge. Alternatively, the beads can be used to make an affinity column. In such cases, the library is passed through the column one or more times to permit binding. The column then is washed to remove nonbinding library members. Magnetic beads are essentially a variant on the above; the target is attached to magnetic beads which are then used in the selection.

There are many reactive matrices available for immobilizing the target molecule, including matrices bearing -NH₂ groups or -SH groups. The target molecule can be immobilized by conjugation with NHS ester or maleimide groups covalently linked to Sepharose beads and the integrity of known properties of the target molecule can be verified. Activated beads are available with attachment sites for -NH₂ or -COOH groups (which can be used for coupling). Alternatively, the target molecule is blotted onto nitrocellulose or PVDF. When using a blotting strategy, the blot should be blocked (*e*.*g.*, with BSA or similar protein) after immobilization of the target to prevent nonspecific binding of library members to the blot.

Library members that bind a target molecule can be released by denaturation, acid, or chaotropic salts. Alternatively, elution conditions can be more specific to reduce background or to select for a desired specificity. Elution can be accomplished using proteolysis to cleave a linker between the target molecule and the immobilizing surface or between the reaction product and the template. Also, elution can be accomplished by competition with a known competitive ligand for the target molecule. Alternatively, a PCR reaction can be performed directly in the presence of the washed target molecules at the end of the selection procedure. Thus, the binding molecules need not be elutable from the target to be selectable since only the template is needed for further amplification or cloning, not the reaction product itself. Indeed, some target molecules bind the most avid ligands so tightly that elution would be difficult.

To select for a molecule that binds a protein expressible on a cell surface, such as an ion channel or a transmembrane receptor, the cells themselves can be used as the selection agent. The library preferably is first exposed to cells not expressing the target molecule on their surfaces to remove library members that bind specifically or non specifically to other cell surface epitopes. Alternatively, cells lacking the target molecule are present in large excess in the selection process and separable (by fluorescence-activated cell sorting (FACS), for example) from cells bearing the target molecule. In either method, cells bearing the target molecule then are used to isolate library members bearing the target molecule (*e*.*g*., by sedimenting the cells or by FACS sorting). For example, a recombinant DNA encoding the target molecule can be introduced into a cell line; library members that bind the transformed cells but not the untransformed cells are enriched for target molecule binders. This approach is also called subtraction selection and has successfully been used for phage display on antibody libraries (Hoogenboom et al. (1998) IMMUNOTECH 4: 1- 20).

A selection procedure can also involve selection for binding to cell surface receptors that are internalized so that the receptor together with the selected binding molecule passes into the cytoplasm, nucleus, or other cellular compartment, such as the Golgi or lysosomes. Depending on the dissociation rate constant for specific selected binding molecules, these molecules may localize primarily within the intracellular compartments. Internalized library members can be distinguished from molecules attached to the cell surface by washing the cells, preferably with a denaturant. More preferably, standard subcellular fractionation techniques are used to isolate the selected library members in a desired subcellular compartment.

An alternative selection protocol also includes a known, weak ligand affixed to each member of the library. The known ligand guides the selection by interacting with a defined part of the target molecule and focuses the selection on molecules that bind to the same region, providing a cooperative effect. This can be particularly useful for increasing the affinity of a ligand with a desired biological function but with too low a potency.

Other methods for selection or partitioning are also available for use with the present invention. These include, for example: immunoprecipitation (direct or indirect) where the target molecule is captured together with library members; mobility shift assays in agarose or polyacrylamide gels, where the selected library members migrate with the target molecule in a gel; cesium chloride gradient centrifugation to isolate the target molecule with library members; mass spectroscopy to identify target molecules labeled with library members. In general, any method where the library member/ target molecule complex can be separated from library members not bound to the target is useful.

The selection process is well suited for optimizations, where the selection steps are made in series, starting with the selection of binding molecules and ending with an optimized binding molecule. The procedures in each step can be automated using various robotic systems. Thus, the invention permits supplying a suitable library and target molecule to a fully automatic system which finally generates an optimized binding molecule. Under ideal conditions, this process should run without any requirement for external work outside the robotic system during the entire procedure.

The selection methods of the present invention can be combined with secondary selection or screening to identify reaction products capable of modifying target molecule function upon binding. Thus, the methods described herein can be employed to isolate or produce binding molecules that bind to and modify the function of any protein or nucleic acid. For example, nucleic acid-templated chemistry can be used to identify, isolate, or produce binding molecules (1) affecting catalytic activity of target enzymes by inhibiting catalysis or modifying substrate binding; (2) affecting the functionality of protein receptors, by inhibiting binding to receptors or by modifying the specificity of binding to receptors; (3) affecting the formation of protein multimers by disrupting the quaternary structure of protein subunits; or (4) modifying transport properties of a protein by disrupting transport of small molecules or ions.

Functional assays can be included in the selection process. For example, after selecting for binding activity, selected library members can be directly tested for a desired functional effect, such as an effect on cell signaling. This can, for example, be performed via FACS methodologies.

The binding molecules of the invention can be selected for other properties in addition to binding. For example, to select for stability of binding interactions in a desired working environment. If stability in the presence of a certain protease is desired, that protease can be part of the buffer medium used during selection. Similarly, the selection can be performed in serum or cell extracts or in any type of medium, aqueous or organic. Conditions that disrupt or degrade the template should however be avoided to allow subsequent amplification.

### (iv) Other Selections

Selections for other desired properties, such as catalytic or other functional activities, can also be performed. Generally, the selection should be designed such that library members with the desired activity are isolatable on that basis from other library members. For example, library members can be screened for the ability to fold or otherwise significantly change conformation in the presence of a target molecule, such as a metal ion, or under particular pH or salinity conditions. The folded library members can be isolated by performing non-denaturing gel electrophoresis under the conditions of interest. The folded library members migrate to a different position in the gel and can subsequently be extracted from the gel and isolated.

Similarly, reaction products that fluoresce in the presence of specific ligands may be selected by FACS based sorting of translated polymers linked through their DNA templates to beads. Those beads that fluoresce in the presence, but not in the absence, of the target ligand are isolated and characterized. Useful beads with a homogenous population of nucleic acid-templates on any bead can be prepared using the split-pool synthesis technique on the bead, such that each bead is exposed to only a single nucleotide sequence. Alternatively, a different anti-template (each complementary to only a single, different template) can by synthesized on beads using a split-pool technique, and then can anneal to capture a solution-phase library.

Biotin-terminated biopolymers can be selected for the actual *catalysis* of bond-breaking reactions by passing these biopolymers over a resin linked through a substrate to avidin. Those biopolymers that catalyze substrate cleavage self-elute from a column charged with this resin. Similarly, biotin-terminated biopolymers can be selected for the catalysis of bond-forming reactions. One substrate is linked to resin and the second substrate is linked to avidin. Biopolymers that catalyze bond formation between the substrates are selected by their ability to react the substrates together, resulting in attachment of the biopolymer to the resin.

Library members can also be selected for their catalytic effects on synthesis of a polymer to which the template is or becomes attached. For example, the library member may influence the selection of monomer units to be polymerized as well as how the polymerization reaction takes place (*e*.*g*., stereochemistry, tacticity, activity). The synthesized polymers can be selected for specific properties, such as, molecular weight, density, hydrophobicity, tacticity, stereoselectivity, using standard techniques, such as, electrophoresis, gel filtration, centrifugal sedimentation, or partitioning into solvents of different hydrophobicities. The attached template that directed the synthesis of the polymer can then be identified.

Library members that catalyze virtually any reaction causing bond formation between two substrate molecules or resulting in bond breakage into two product molecules can be selected using the schemes proposed herein. To select for bond forming catalysts (for example, hetero Diels-Alder, Heck coupling, aldol reaction, or olefin metathesis catalysts), library members are covalently linked to one substrate through their 5' amino or thiol termini. The other substrate of the reaction is synthesized as a derivative linked to biotin. When dilute solutions of library-substrate conjugate are combined with the substrate-biotin conjugate, those library members that catalyze bond formation cause the biotin group to become covalently attached to themselves. Active bond forming catalysts can then be separated from inactive library members by capturing the former with immobilized streptavidin and washing away inactive library members

In an analogous manner, library members that catalyze bond cleavage reactions such as retro-aldol reactions, amide hydrolysis, elimination reactions, or olefin dihydroxylation followed by periodate cleavage can be selected. In this case, library members are covalently linked to biotinylated substrates such that the bond breakage reaction causes the disconnection of the biotin moiety from the library members. Upon incubation under reaction conditions, active catalysts, but not inactive library members, induce the loss of their biotin groups. Streptavidin-linked beads can then be used to capture inactive polymers, while active catalysts are able to be eluted from the beads. Related bond formation and bond cleavage selections have been used successfully in catalytic RNA and DNA evolution (Jäschke et al. (2000) CURR. OPIN. CHEM. BIOL. 4: 257-62) Although these selections do not explicitly select for multiple turnover catalysis, RNAs and DNAs selected in this manner have in general proven to be multiple turnover catalysts when separated from their substrate moieties (Jäschke et al. (2000) CURR. OPIN. CHEM. BIOL. 4: 257-62; Jaeger et al. (1999) PROC. NATL. ACAD. SCI. USA 96: 14712-7; Bartel et al. (1993) SCIENCE 261: 1411-8; Sen et al. (1998) CURR. OPIN. CHEM. BIOL. 2: 680-7).

In addition to simply evolving active catalysts, the *in vitro* selections described above are used to evolve non-natural polymer libraries in powerful directions difficult to achieve using other catalyst discovery approaches. Substrate specificity among catalysts can be selected by selecting for active catalysts in the presence of the desired substrate and then selecting for *inactive* catalysts in the presence of one or more undesired substrates. If the desired and undesired substrates differ by their configuration at one or more stereocenters, enantioselective or diastereoselective catalysts can emerge from rounds of selection. Similarly, metal selectivity can be evolved by selecting for active catalysts in the presence of desired metals and selecting for inactive catalysts in the presence of undesired metals. Conversely, catalysts with broad substrate tolerance can be evolved by varying substrate structures between successive rounds of selection.

Importantly, *in vitro* selections can also select for specificity in addition to binding affinity. Library screening methods for binding specificity typically require duplicating the entire screen for each target or non-target of interest. In contrast, selections for specificity can be performed in a single experiment by selecting for target binding as well as for the inability to bind one or more non-targets. Thus, the library can be pre-depleted by removing library members that bind to a non-target. Alternatively, or in addition, selection for binding to the target molecule can be performed in the presence of an excess of one or more non-targets. To maximize specificity, the non-target can be a homologous molecule. If the target molecule is a protein, appropriate non-target proteins include, for example, a generally promiscuous protein such as an albumin. If the binding assay is designed to target only a specific portion of a target molecule, the non-target can be a variation on the molecule in which that portion has been changed or removed.

### (vi) Amplification and Sequencing

Once all rounds of selection are complete, the templates which are, or formerly were, associated with the selected reaction product preferably are amplified using any suitable technique to facilitate sequencing or other subsequent manipulation of the templates. Natural oligonucleotides can be amplified by any state of the art method. These methods include, for example, polymerase chain reaction (PCR); nucleic acid sequence-based amplification (see, for example, Compton (1991) NATURE 350: 91-92), amplified anti-sense RNA (see, for example, van Gelder et al. (1988) PROC. NATL. ACAD. SCI. USA 85: 77652-77656); self-sustained sequence replication systems (Gnatelli et al. (1990) PROC. NATL. ACID. SCI. USA 87: 1874-1878); polymerase-independent amplification (see, for example, Schmidt et al. (1997) NUCLEIC ACIDS RES. 25: 4797-4802, and *in vivo* amplification of plasmids carrying cloned DNA fragments. Descriptions of PCR methods are found, for example, in Saiki et al. (1985) SCIENCE 230: 1350-1354; Scharf et al. (1986) SCIENCE 233: 1076-1078; and in U.S. Patent No. 4,683,202. Ligase-mediated amplification methods such as Ligase Chain Reaction (LCR) may also be used. In general, any means allowing faithful, efficient amplification of selected nucleic acid sequences can be employed in the method of the present invention. It is preferable, although not necessary, that the proportionate representations of the sequences after amplification reflect the relative proportions of sequences in the mixture before amplification.

For non-natural nucleotides the choices of efficient amplification procedures are fewer. As non-natural nucleotides can be incorporated by certain enzymes including polymerases it will be possible to perform manual polymerase chain reaction by adding the polymerase during each extension cycle.

For oligonucleotides containing nucleotide analogs, fewer methods for amplification exist. One may use non-enzyme mediated amplification schemes (Schmidt et al. (1997) NUCLEIC ACIDS RES. 25: 4797-4802). For backbone-modified oligonucleotides such as PNA and LNA, this amplification method may be used. Alternatively, standard PCR can be used to amplify a DNA from a PNA or LNA oligonucleotide template. Before or during amplification the templates or complementing templates may be mutagenized or recombined in order to create an evolved library for the next round of selection or screening.

### (vii) Sequence Determination and Template Evolution

Sequencing can be done by a standard dideoxy chain termination method, or by chemical sequencing, for example, using the Maxam-Gilbert sequencing procedure. Alternatively, the sequence of the template (or, if a long template is used, the variable portion(s) thereof) can be determined by hybridization to a chip. For example, a single-stranded template molecule associated with a detectable moiety such as a fluorescent moiety is exposed to a chip bearing a large number of clonal populations of single-stranded nucleic acids or nucleic acid analogs of known sequence, each clonal population being present at a particular addressable location on the chip. The template sequences are permitted to anneal to the chip sequences. The position of the detectable moieties on the chip then is determined. Based upon the location of the detectable moiety and the immobilized sequence at that location, the sequence of the template can be determined. It is contemplated that large numbers of such oligonucleotides can be immobilized in an array on a chip or other solid support.

Libraries can be evolved by introducing mutations at the DNA level, for example, using error-prone PCR (Cadwell et al. (1992) PCR METHODS APPL. 2: 28) or by subjecting the DNA to *in vitro* homologous recombination (Stemmer (1994) PROC. NATL. ACAD. SCI. USA 91: 10747; Stemmer (1994) NATURE 370: 389) or by cassette mutagenesis.

### (a) Error-prone PCR

Random point mutagenesis is performed by conducting the PCR amplification step under error-prone PCR (Cadwell et al. (1992) PCR METHODS APPLIC. 2: 28-33) conditions. Because the genetic code of these molecules are written to assign related codons to related chemical groups, similar to the way that the natural protein genetic code is constructed, random point mutations in the templates encoding selected molecules will diversify progeny towards chemically related analogs. Because error-prone PCR is inherently less efficient than normal PCR, error-prone PCR diversification is preferably conducted with only natural dATP, dTTP, dCTP, and dGTP and using primers that lack chemical handles or biotin groups.

### (b) Recombination

Libraries may be diversified using recombination. For example, templates to be recombined may have a structure in which codons are separated by five-base non-palindromic restriction endonuclease cleavage sites such as those cleaved by *Ava*II (G/GWCC, W=A or T), *Sau*96I (G/GNCC, N=A, G, T, or C), *Dde*I (C/TNAG), or *Hin*FI (G/ANTC). Following selections, templates encoding desired molecules are enzymatically digested with these commercially available restriction enzymes. The digested fragments then are recombined into intact templates with T4 DNA ligase. Because the restriction sites separating codons are nonpalindromic, template fragments can *only* reassemble to form intact recombined templates. DNA-templated translation of recombined templates provides recombined small molecules. In this way, functional groups between synthetic small molecules with desired activities are recombined in a manner analogous to the recombination of amino acid residues between proteins in Nature. It is well appreciated that recombination explores the sequence space of a molecule much more efficiently than point mutagenesis alone (Minshull et al. (1999) CURR. OPIN. CHEM. BIOL. 3: 284-90; Bogarad et al. (1999) PROC. NATL. ACAD. SCI. USA 96: 2591-5; Stemmer NATURE 370: 389-391).

A preferred method of diversifying library members is through nonhomologous random recombination, as described, for example, in WO 02/074978; US Patent Application Publication No. 2003-0027180-A1; and Bittker et al. (2002) NATURE BIOTECH. 20(10): 1024-9.

### (c) Random Cassette Mutagenesis

Random cassette mutagenesis is useful to create a diversified library from a fixed starting sequence. Thus, such a method can be used, for example, after a library has been subjected to selection and one or more library members have been isolated and sequenced. Generally, a library of oligonucleotides with variations on the starting sequence is generated by traditional chemical synthesis, error-prone PCR, or other methods. For example, a library of oligonucleotides can be generated in which, for each nucleotide position in a codon, the nucleotide has a 90% probability of being identical to the starting sequence at that position, and a 10% probability of being different. The oligonucleotides can be complete templates when synthesized, or can be fragments that are subsequently ligated with other oligonucleotides to form a diverse library of templates.

The following examples contain important additional information, exemplification and guidance that can be adapted to the practice of this invention in its various embodiments and equivalents thereof. Practice of the invention will be more fully understood from these following examples, which are presented herein for illustrative purpose only, and should not be construed as limiting in anyway.

### EXAMPLES

### Example 1. Ordered Multi-step Triolefin Sequence Synthesis in a Single Solution Directed by DNA Templates

This example describes the ordered multi-step syntheses of a triolefin using DNA-linked substrates of comparable intrinsic reactivity that are simultaneously present in one solution.

*General Synthesis and Analysis Methods*. DNA oligonucleotides were synthesized on a PerSeptive Biosystems Expedite 8090 DNA synthesizer using standard phosphoramidite protocols and purified by reverse phase HPLC using a triethylammonium acetate (TEAA)/CH₃CN gradient. Modified phosphoramidites and CPG for DNA synthesis were purchased from Glen Research (Sterling, VA). The 5'-amino modified oligonucleotides were synthesized using the 5'-amino modifier 5 phosphoramidite. The 3'-amino modified oligonucleotides were synthesized using 3'-Amino-Modifier C7 CPG 500. The 3'-thiol modified oligonucleotides were synthesized using 3'-Thiol-Modifier C3 S-S CPG. The 5'-thiol modified oligonucleotides were synthesized using 5'-Thiol Modifier C6. The doubly biotinylated (3' and 5'-biotin modified) oligonucleotides were synthesized using 3'-BiotinTEG-CPG to install the 3'-biotin group and 5'-Biotin Phosphoramidite to install the 5'-biotin group. Oligonucleotides were quantitated by UV and all modified DNAs and reagents were characterized by MALDI-TOF mass spectrometry. Reaction products, including multi-step reaction sequences, were also characterized by MALDI-TOF mass spectrometry as described below. Reaction yields were characterized by denaturing polyacrylamide gel electrophoresis (PAGE) followed by ethidium bromide staining, UV visualization, and CCD-based densitometry. All chemicals, unless otherwise noted, were purchased from Sigma-Aldrich.

*Oligonucleotide Sequences.* The oligonucleotides used in this experiment included:
Reagent 1/1b: 5'-CGACTGTGA-NH₂ (SEQ ID NO: 1)
Mismatched 1c: 5'-CTCTGGTGA-NH₂ (SEQ ID NO: 2)
Reagent 2/2b: 5'-H₂N-GGACAACATGTG (SEQ ID NO: 3)
Mismatched 2c: 5'-H₂N-GGTGACAATCTG (SEQ ID NO: 4)
Reagent 3/3b: 5'-GGGGCTGACGGGCTATCGCTTGTGA-NH₂ (SEQ ID NO: 5)
Mismatched 3c: 5'-GGGTCCGTCCGCCAATCTCTCGTGA-NH₂ (SEQ ID NO: 6)
Template 4: 5'-H₂N-TCACATGTTGTCCATCACAGTCGTAGCGATAGCCCGTCAGCCCC (SEQ ID NO: 7)
Complementary oligonucleotide for restriction digestion and MALDI analysis of products linked to template 4: 5'-CTGTGATGGACAACATGTGA (SEQ ID NO: 8)

*Preparation of Phosphorane Reagents **1, 2**, and **3**.* Reagent oligonucleotides were synthesized as described above on CPG resin with either a 3'-amino modification (for **1, 3**) or a 5'-amino modification (for **2**). Oligonucleotides with 3'-amino termini were treated with piperidine:DMF (20:80) to deprotect the amino group; 5'-amino modified oligonucleotides were synthesized without the terminal MMT group then washed with DIPEA in DMF. To CPG resin linked to these oligonucleotides was added 20 mg N-(3-dimethylaminopropyl)-N'-ethylcarbodiimide hydrochloride (EDC; ∼100 µmol) and 30 mg 4-(diphenylphosphino)benzoic acid (∼100 µmol) in 500 µL dry DMF with 50 µL DIPEA. The mixture was incubated at 37 °C for > 4 hours. The beads were washed with DMF, deprotected and cleaved in 1:1 concentrated ammonium hydroxide:aqueous methyl amine supplemented with 4 mg/mL tris (2-carboxyethyl)phosphine hydrochloride (TCEP) at 65 °C for 10 minutes, purified by reverse-phase HPLC, and lyophilized.

The phosphine-linked oligonucleotides were redissolved in 0.2 M sodium phosphate buffer, pH 7.2, and combined with 2 mg/mL N-succinimidyl(4-iodoacetyl)aminobenzoate (SIAB, Pierce) for 5 minutes before the addition of an appropriate amine-containing reagent. For 1, this reagent is biocytin (Bachem) which was added in slight excess relative to the SIAB linker and reacted for 90 minutes at 25 °C.

For **2/2b** and **3/3b,** the additional reagent is a tartrate-modified amine, prepared as follows. A diamine (100 µmol, ethylenediamine for R³, 1,3-diaminopropane for R², 1,8-diaminooctane for a control substrate below) in CH₂Cl₂ was added dropwise to a solution of 33 mg diacetyl tartaric anhydride (150 µmol) in 1 mL CH₂Cl₂. After 1 hour at 25 °C, 1 N NaOH was added and the reaction stirred for 1 hour to fully cleave the acetate protecting groups. HCl as added to neutralize the solution and the aqueous layer was recovered and concentrated in *vacuo*. (For 1,8-diaminooctane, the organic layer was recovered instead). One-tenth of this crude reaction, dissolved in 1:1 DMF:0.2 M sodium phosphate, pH 7.2, was added to the oligonucleotide/SIAB mixture described above and reacted for 90 minutes after which 1 µL of acetic anhydride was added.

For all reagents, the reactions were desalted by gel filtration using Sephadex G-25 and purified by reverse-phase HPLC. All phosphorane reagents were characterized by MALDI-TOF mass spectrometry. The tartrate-modified reagents are **2b** and **3b** respectively; aldehydes **2** and **3** are made from **2b** and **3b** by NaIO₄ oxidation immediately prior to use.

*Preparation of Aldehyde Template 4.* The template was synthesized with a 5'-amino modification (MMT off) and then washed with DIPEA in DMF to deprotonate the amines. Diacetyl tartaric anhydride (21.6 mg, 100 µmol), 15.3 mg HOBt (100 µmol), and 16.0 mg tryptamine (100 µmol) were mixed together in 400 µL dry DMF for 1 hour. A solution of 20.6 mg 1,3-dicyclohexylcarbodiimide (100 µmol) in 100 µL DMF was added and the resulting solution was incubated at 25 °C for 30 minutes. The solution was centrifuged and the supernatant added to the CPG beads. After 2 hours, the beads were washed, deprotected/cleaved from the support with 1:1 ammonium hydroxide:methyl amine for 10 minutes at 65 °C, and purified by reverse-phase HPLC. Following lyophilization, the collected tartrate-modified template was redissolved in 0.05 M NaOAc, pH 5.0, and oxidized using 50 mM NaIO₄ for 45 minutes. The reaction was desalted by gel filtration using Sephadex G-25 and purified by reverse-phase HPLC to yield the aldehyde template **4,** verified by MALDI-TOF mass spectrometry.

*Stability Of Phosphorane Reagents In Solution.* Previous work (Gartner et al. (2004) NATURE 431: 545-549) has shown that intramolecular cyclizations between DNA-linked ylides and aldehydes are possible, forming macrocylic fumaramides. To demonstrate that the phosphorane reagents used for this ordered synthesis could not cyclize intramolecularly and were stable to the reaction conditions, control phosphorane reagents were made (as described above) that could directly react with an aldehyde-linked template. (Gartner et al. (2002) ANGEW. CHEM. INT. ED. 123: 1796-1800) The reagents used either a 1,3-diaminopropane or a 1,8-diaminooctane linker between the ylide and aldehyde groups as shown **(****FIG. 5A****).** The reagents were oxidized with NaIO₄ in 50 mM NaOAc, pH 5.0 and added to 0.1M TAPS buffer pH 8.0 with 1 M NaCl (150 nM reagent concentration). The aldehyde template **4** was added to these reactions either immediately or after 15 minutes, 30 minutes, 1 hour, or 2 hours. The reactions were precipitated with ethanol and analyzed by denaturing PAGE. The product yield for the 1,3-diaminopropane linker did not noticeably decrease after as much as 2 hours of preincubation; however, the product for the 1,8-diaminooctane essentially disappeared after 2 hours **(****FIG. 5B****).** These results suggest that the reagents themselves were stable in solution except when intramolecular cyclization is possible. While some hydrolysis of the ylide was observed, particularly at higher temperatures, the building blocks for the triolefination sequence were stable to the reaction conditions used.

*Testing the Reactivity of Reagent **1** in the Presence on Absence of **2***. To allow the one-step purification of the desired triolefin **5,** the system was designed with a purifiable group (biocytin) attached to **1** so that only a product that has undergone three successive Wittig olefinations would link R¹ to the template (the truncated products template-R³ and template-R³-R² as well as an unreacted template would lack biotin). This system requires that **2,** when present in the reaction, prevents direct reactivity of **1** (and transfer of R¹) to the template **4.** Reactions were performed using the multi-step conditions (1 hour at 4 °C, **1** hour at 30 °C, 2 hours at 60 °C) with varying equivalents of **2** added to **4** (100 nM) in 0.1 M TAPS, pH 8.0 before adding **1** (200 nM, 2 equivalents) to the reaction. Control reactions were performed that lacked **2** entirely or that replaced **2** with a mismatched reagent **2c** (200 nM, 2 equivalents) that cannot anneal to the template. The reactions were precipitated with ethanol and analyzed by denaturing PAGE. The results show that a single full equivalent of **2** blocks the direct reaction of **1** with **4 (****FIG. 6****).**

*Test of Two-Step Transfer*. To demonstrate the proper transfer of the first two reagents onto the third reagent, a model reaction was performed that replaces **3** with a DNA-linked aldehyde **3d** that can stably capture products. This aldehyde was synthesized starting with the synthesis for **3** (described above) and the amine on beads was reacted directly with 1 mg of the NHS ester of carboxybenzaldehyde in DMF followed by deprotection, cleavage, and HPLC purification as described above.

The two-step transfer was performed by oxidizing either **2** or its mismatched variant **(2c)** with 1.5 mM NaIO₄ in 50 mM NaOAc buffer, pH 5.0 for 30 min. An equal volume of 2 M NaCl was added as well as **4** and the solution cooled to 4 °C. One equivalent or either **1** or (mismatched) **1c,** 1 equivalent **3d,** and **1** equivalent **4** were then added to the solution and allowed to hybridize for about 10 minutes. Under the pH 5.0 conditions, no Wittig olefination reactivity was seen (data not shown). The reaction was initiated by adding 150 µL 0.1M TAPS pH 8.0, 1 M NaCl and was incubated for 1 hour at 4 °C then 1 hour at 30 °C before ethanol precipitation. In the final mixtures, all species are present at 100 nM. Identical reactions were performed that use **2b** (the unoxidized tartrate) instead of **2.** Products from the matched reactions of **2/2b** and **1** were isolated using streptavidin-agarose beads (Novagen), washed with H₂O, and eluted by heating at 95 °C with 95% formamide, 10 mM EDTA for 10 minutes before ethanol precipitating. Reactions were analyzed by denaturing PAGE. Only with the matched, aldehyde-terminated reagents **(1** and **2)** was the transfer of the biotin group on R¹ observed **(****FIG. 7****).** The two-step transfer occurred in 47% overall yield.

*Three-Step Ordered Wittig Olefination*. The three-step reaction was set up in an identical manner as the two-step reaction described above but with **3** instead of **3d** and both **2** and **3** oxidized for 30 min with 1.5 mM NaIO₄. The template **4** was hybridized with **1, 2,** and **3** at pH 5.0, 4 °C for 10 minutes. To start the reaction, 0.1 M TAPS pH 8.0, 1 M NaCl was added. In the final mixture, all reagents were at concentrations of 100 nM. To facilitate analysis, control reactions were also performed that use tartrates **2b,** or **2b** and **3b,** in place of their oxidized counterparts to prevent the first step or both the first two steps from occurring. Reactions were run for 1 hour at 4 °C, 1 hour at 30 °C, and 2 hours at 60 °C before ethanol precipitation. Aliquots of the final reaction mixture were purified using streptavidin-agarose (Novagen) as described above and analyzed by denaturing PAGE **(****FIG. 2A****).** Because of the potential of **2** and **3** to react with each other and cyclize if **2** fails to react with **1,** there is no observed diolefin side product in lane C of **FIG. 2A** and more unmodified template is observed compared to lanes A or B despite equivalent amounts of starting material.

Reagents were also synthesized that swapped the building blocks R² and R³ on **2** and **3.** Reactions were run under identical conditions and then analyzed by PAGE. The R²-R³-R¹ product was generated in 15% yield **(****FIG. 8****).**

*MALDI-TOF Mass Spectrometry of Reaction Products*. The samples for the control reactions with unoxidized reagents lacking aldehyde groups (to produce mono/di-olefins) were prepared for MALDI-TOF as follows. The reaction was annealed (70 °C for 5 minutes, 55 °C for 12 minutes, then 37 °C) to a doubly biotinylated complementary oligonucleotide (3 equivalents), captured with streptavidin agarose, washed with TAPS buffer, pH 8.0, 1 M NaCl several times to remove **1, 2,** and **3** from the template, and eluted in 95% formamide, 10 mM EDTA before ethanol precipitation. The pellets are redissolved in NEBuffer 4 (New England Biolabs) and annealed as above before adding BSA and *Nla*III to cleave the template to leave a 7-base sequence suitable for MALDI. After 4 hours, the enzyme was denatured and the digestion products captured with streptavidin-agarose. The supernatant and first H₂O wash were precipitated with ethanol and subjected to MALDI analysis.

The three-step reaction was prepared for MALDI-TOF as follows. The reaction was precipitated with ethanol, redissolved in NEBuffer 4 (New England Biolabs) and 3 equivalents of a nonbiotinylated DNA complement was added. The solution was annealed as above and then BSA and *Nla*III were added for restriction digestion. Following denaturation and streptavidin-agarose purification, the sample was washed several times with H₂O and eluted with 95% formamide, 10 mM EDTA before ethanol precipitation. Only biotinylated templates (with R¹) remain in the analyzed sample.

Samples were redissolved in 0.1M TEAA, purified using Zip-Tips (Millipore) and spotted on a MALDI plate in a 9:1 matrix of 40 mg/mL 2,4,6-trihydroxyacetophenone (THAP, Fluka) in 1:1 ACN: ddH₂O to 50 mg/mL ammonium citrate (dibasic) in ddH₂O. The resulting spectra are present in **FIGS. 2B** and **2C****.** The predicted and observed masses for all possible products are presented in **Table 2.** The prime designation (R2' and R3') refers to the unoxidized tartrate form of these building blocks from **2b** or **3b.** The template **4** has been digested to a 7-mer prior to analysis by *Hla*III. Because of potential ionization differences between product species, the relative heights of peaks included in MALDI spectra may not be representative of the relative amounts of individual species in the product mixtures.

**Table 2: Summary of predicted and observed masses by MALDI-TOF mass spectroscopy of olefination products.**

| Species | Predicted Mass | Observed Mass |
|---|---|---|
| Template-Aldeliyde (from 4) | 2302.42 (2319.4 for hydrated form) | (2316.3 to 2315.4) ± 6 (Fig. 2b, 2c; left and middle) |
| Template-R³ | 2637.55 | 2636.5 ± 6 (Fig. 2b, left) |
| Template-R² | 2651.55 | 2652.6 ±6 6 (Fig. 2c, left) |
| Template-R¹ | 2817.64 | 2814.6 ± 6 (Fig. 2b, right) |
| Template-R³-R^{2'} Template-R²-R^{3'} | 2910.66 | 2906.0 ± 6 (Fig. 2b, middle) 2909.6 ± 6 (Fig. 2c, middle) |
| Template-R³-R¹ | 3076.74 | Not observed |
| Template-R²-R¹ | 3090.75 | 3090.0 ± 6 (Fig. 2c, right) |
| Template-R³-R¹-R¹ Template-R²-R³-R¹ | 3349.55 | 3347.9 ± 6 (Fig. 2b, right) 3350.6 ± 6 (Fig. 2c, right) |

*Mismatch Triolefin Controls*. Reagents **1c, 2c, 3c** were prepared identically to **1, 2,** and **3** but with scrambled oligonucleotide sequences that cannot anneal to template **4.** The three-step sequence described above was performed three additional times with one of each of the mismatch reagents **(1c, 2c,** or **3c**) replacing one of the original reagents **(1, 2,** or **3,** respectively). The reactions were analyzed directly as well as after streptavidin purification by denaturing PAGE. For **1c,** reagent **2** and **3** (each with an ylide and aldehyde) can react twice with each other to release their attached substrates by cyclization, thereby transferring no material and no R¹ to the template. For **2c,** reagent **1** and **3** can react directly with **4** and a one-step biotinylation product was recovered. For **3c,** no material was transferred to the template **4 (****FIG. 9****).** The results shown in **FIG. 9** demonstrate that all three reagents must be capable of hybridizing to the template to produce the multistep product. These observations are consistent with the sequence selectivity of these DNA-templated reactions.

*DNA-Linked NHS Interferes with Reagent Transfer*. To test whether the strategy in **FIG. 1** could be extended to the production of a tripeptide using NHS-linked DNAs, NHS-linked oligonucleotide reagents **1n** and **2n** were synthesized using the same DNA sequence as those for **1** and **2** but with 3' and 5' thiol modifications, respectively.

The protected thiol oligonucleotides were purified following DNA synthesis using reverse-phase HPLC. For the 3' thiol **(1n),** the DMT group was deprotected in 3% TFA and precipitated with ethanol. To deprotect the thiol group, 100mM DTT, pH 8.5 was added at 25 °C for 30 minutes. The reaction was desalted by gel filtration using Sephadex G-25 and added directly to 250 µL of 40 mg/mL solution of N-hydroxymaleimide in 0.5M MOPS, pH 7.5. After 30 minutes, the reaction was concentrated *in vacuo*, desalted by gel filtration, and purified by reverse-phase HPLC to generate a 3' NHS-linked oligonucleotide. For the 5' thiol **(2n),** the oligonucleotide was redissolved in 200 µL 0.1 M TEAA, pH 7.0. 75 µL of 1M AgNO₃ was added and after 30 minutes, 75 µL of 1.33 M DTT was added. The solution was centrifuged and the supernatant collected. The pellet was washed with 150 µL 0.1 M TEAA and, after centrifugation, the supernatant was collected again. The combined supernatants were desalted by gel filtration using Sephadex G-25 and added directly to 250 µL of a 40 mg/mL solution of N-hydroxymaleimide in 0.5 M MOPS, pH 7.5. After 30 minutes, the reaction was concentrated *in vacuo*, desalted by gel filtration, and purified by reverse phase HPLC to generate 5' NHS- linked oligonucleotide.

Biotin was activated as an NHS-ester on **In** by adding approximately 0.5 mg of biotin in DMF to 0.2 mg of EDC (total volume approximately 50 µL). After 20 minutes, 20 µL of this mixture was added directly to an aliquot of NHS-linked oligonucleotide in 80 µL 0.1 M MES pH 6.0. After 5 minutes, the reaction was desalted by gel filtration using Sephadex G-25 and purified by reverse-phase HPLC. By adding 1% TFA to the collected material in TEAA:CH₃CN before lyophilization, the NHS ester-linked DNA could be recovered in pure form (without the addition of TFA, hydrolysis of the NHS ester occurs under the lyophilization conditions).

To test the ability of NHS to attack and capture NHS-linked esters, an experiment was performed with unmodified DNA **4b** (same sequence as **4),** 3'-amine modified **3e** (same sequence as **3),** and **1n-biotin** and **2n (****FIG. 10****).** Species **1n-biotin, 2n, 3e,** and **4b** were annealed together at 100 nM in 0.1 M MOPS, pH 7.0, 1 M NaCl, 4 °C. The reaction was run for 20 minutes at 4 °C and 20 minutes at 30 °C before being precipitated with ethanol or purified by streptavidin-agarose beads as described above. A control reaction excluding **2n** was also performed.

Denaturing PAGE analysis of the reactions demonstrated that biotin can be transferred from **1n** to **3e** only in the presence of **2n;** this strongly suggests that the NHS group in **2n** can reversibly capture proximal NHS-linked esters **(****FIG. 10****).** A tripeptide synthesis using the method in **FIG. 1** would be problematic as consumed reagents (DNA-linked NHS groups) would sometimes capture growing product molecules due to this reversible transfer of esters between NHS groups.

### Example 2. Template Masking To Control Reactivity

To demonstrate that oligonucleotide masks such as **10** and **11** can be used to control the reactivity of DNA-linked reagents, three DNA-linked phosphoranes were synthesized **(6, 7, 8;** Gartner et al. (2001) J. AM. CHEM. Soc. 123: 6961-6963), as well as an aldehyde-linked template **9** as previously described. (Gartner et al. (2002) ANGEW. CHEM. INT. ED. 123: 1796-1800). The oligonucleotides used in this experiment included:
Reagent **6:** 5'-CATGAGAAC-NH₂ (SEQ ID NO: 9)
Reagent **7:** 5'-CTGTGATGGACCAGAAC-NH₂ (SEQ ID NO: 10)
Reagent **8:** 5'-CTGACGGGCTATCGCTACGAAGAAC-NH₂ (SEQ ID NO: 11)
Template **9:** 5'-H₂N-GTTCTCATGGTCCATCACAGTCGTAGCGATAGCCCGTCAG (SEQ ID NO: 12)
Mask **10:** 5'-TGTGATGG (SEQ ID NO: 13)
Mask **11:** 5'-ACGGGCTATCGCTACG (SEQ ID NO: 14)

The reaction schemes are summarized in **FIG 3A****.** The template **9** (at 150 nM) and masks **10** and **11** (at 225 nM) were preannealed in 0.1 M TAPS, pH 8.0, 1 M NaCl, and then transferred to 4 °C, 25 °C, 42 °C, 57 °C, or 72 °C. An equimolar mixture (200 nM in each reagent after addition) of the three phosphorane reagents **(6, 7,** and **8**) was added and the mixture reacted for 1 hour before ethanol precipitation. Identical reactions were performed that excluded masks **10** and **11.** Denaturing PAGE analysis of the reactions demonstrated that, while all three reagents can react at low temperatures without **10** and **11,** only **6** reacts well in the presence of the masks. As temperature increases, the reactivity of **7** is restored and predominates at 42° C. At the highest temperature (72 °C), only **8** can anneal and react **(****FIG. 3B****).**

### Example 3. Ordered Multi-step tripeptide Sequence Synthesis in a Single Solution Directed by DNA Templates

This example describes the ordered multi-step syntheses of a tripeptide **(****FIG. 4A****)** using DNA-linked substrates of comparable intrinsic reactivity that are simultaneously present in one solution. This example shows that it is possible to perform a single-solution synthesis of an ordered tripeptide using oligonucleotide masks.

*Oligonucleotide Sequences*. The oligonucleotides used in this experiment included:
Template **12:** 5'-H₂N-GTTCTCATGGTCCATCACAGTCGTAGCGATAGCCCGTCAG (SEQ ID NO: 15)
Reagent **13:** 5'-CATGAGAAC-SH (SEQ ID NO: 16)
Mismatched **13b:** 5'-GAACAGAAC-SH (SEQ ID NO: 17)
Reagent **14:** 5'-CTGTGATGGACCAGAAC-SH (SEQ ID NO: 18)
Mismatched **14b:** 5'-CTGCAAAGACGCAGAAC-SH (SEQ ID NO: 19)
Reagent **15:** 5'-CTGACGGGCTATCGCTACGAAGAAC-SH (SEQ ID NO: 20)
Complimentary oligonucleotide for restriction digestion and MALDI analysis of products linked to template **12:** 5'-CTGTGATGGACCATGAGAAC (SEQ ID NO: 21)
Template **12** required no further modifications and was purified directly using reverse-phase HPLC.

*NHS Ester Reagent Preparation*. NHS-linked DNAs were prepared as described above. Briefly, protected 3' thiol oligonucleotides were purified following DNA synthesis using reverse-phase HPLC. Following deprotection of the DMT group in 3% TFA and ethanol precipitation, the oligonucleotides were redissolved in ddH₂O. To deprotect the thiol group, 100 mM DTT, pH 8.5 was added at 25 °C for 30 minutes. The reaction was desalted by gel filtration using Sephadex G-25 and added directly to 250 µL of a 40 mg/mL solution of N-hydroxymaleimide in 0.5 M MOPS, pH 7.5. After 30 minutes, the reaction was concentrated in *vacuo*, desalted by gel filtration, and purified by reverse-phase HPLC to generate NHS-linked oligonucleotides.

NVOC-labeled amino acids were prepared using a previously described protocol (Robertson et al. (1991) J. AM. CHEW. Soc. 113: 2722-2729). Briefly, to a mixture of the amino acid and Na₂CO₃ in H₂O, an equimolar amount of 4,5-dimethoxy-2-nitrobenzyl chloroformate was added in dioxane. After 1 hour at 25 °C, the reaction was quenched with NaHSO₄ and extracted using ethyl acetate. The crude reactions were used directly for labeling of DNA. Amino-terminated oligonucleotides conjugated to the amino acids (using EDC/NHS coupling) and purified by reverse-phase HPLC were characterized by MALDI-TOF. (Gartner et al. (2001) J. AM. CHEM. Soc. 123: 6961-6963.) Exposure of these oligonucleotides to long wavelength (365 nm) UV light for 30 minutes followed resulted in quantitative deprotection as followed by MALDI-TOF.

DNA-linked NHS esters were synthesized as described above using approximately 0.5 mg of either biotin (for **15**) or the crude NVOC-amino acids derived from 4-transaminocrotonic acid (for **14**) or trans-4-(aminomethyl)-cyclohexanecarboxylic acid (for **13**); coupling yields for these reagents ranged from 50-95%. To prepare unprotected DNA-linked NHS esters **13** and **14,** the NVOC-protected NHS ester reagents were dissolved in 50 mM NaOAc pH 5.0, 1 M NaCl and exposed to 365 nm UV light for 30 minutes at 4 °C using a hand-lamp. The deprotections proceed in 90-100% yield; to obtain pure **13** and **14,** the reagents were repurified by reverse-phase HPLC and lyophilized (with 1% TFA).

*Reactivity of DNA-Linked NHS Ester Reagents*. To test the reactivity of the amino acid-linked reagents **13** and **14,** sample reactions were performed by preannealing template **12** (100 nM) and masks **10** and **11** (150 nM) and then adding either approximately 1.0 equivalent or approximately 3.6 equivalents of **13** in 0.1 M MOPS pH 7.0, 1 M NaCl at both 4 °C and 25 °C for 1 hour. The reactions were quenched by the addition of 1 M Tris, pH 8.0 and precipitated with ethanol. The reactions were prepared for MALDI as described above for the Wittig olefination products using a doubly-biotinylated complementary oligonucleotide and analyzed. The MALDI spectra are shown below **(****FIG. 11****).** So long as the temperature is significantly lower than the Tₘ of the reagent, exchange of the reagents does not occur and only a single addition of the amino acid to **12** is seen even with excess reagent. However, at temperatures near the Tₘ, exchange of the reagents allows for multiple additions of the same amino acid. For the purposes of an ordered synthesis, only a single equivalent of the building block is desired so low temperature (non-exchanging) conditions are used. This experiment was repeated for reagent **14** and multiple additions were observed after 1 hour at and above 42 °C but a single addition of R², even with ≥3 equiv. of **14,** is seen at or below 37 °C.

*Three-Step Ordered Tripeptide Synthesis*. Reagents **13, 14,** and **15** were prepared as described, redissolved after lyophilization in 50 mM NaOAc, 1 M NaCl, desalted by gel filtration using Sephadex G-25, and quantitated by UV. Template **12** and masks **10, 11** (1.5 equivalents relative to template) are annealed together at 4 °C in 0.2 M MOPS, pH 7.0, 2 M NaCl. **13** (1.05 equivalents), **14** (1.05 equivalents), **15** (3 equivalents) were added to the reaction mixture and reacted at 4 °C for 20 minutes, 37 °C for 20 minutes, 62 °C for 2 hours. After dilution caused by the addition of the reagents, the final concentration of solutes in this reaction was 0.1 M MOPS, pH 7.0, 1 M NaCl with 100 nM template **12.** Following the 4 °C and 37 °C steps, aliquots of the reaction were quenched by the addition of 1 M Tris, pH 8.0. The final reaction mixture was isolated by streptavidin-linked bead capture and quantitated by denaturing PAGE to give 45% yield of recovered biotinylated template (this yield includes the tripeptide product as well as all truncated side products). The quenched aliquots and final reaction were prepared for MALDI as previously described with the results shown in **FIG 4B****.** A summary of expected masses of DNA-linked peptide products is provided below **(Table 3).** Because of potential ionization differences between product species, the relative heights of the peaks in the included MALDI spectra may not be representative of the relative amounts of individual species in the product mixtures. The template **12** has been digested by *Hla*III to a 9-mer prior to analysis.

**Table 3: Summary of predicted masses for expected peptide products (in increasing order).**

| Species | Predicted Mass | Observed Mass |
|---|---|---|
| Template-NH₃ (from **12**) | 2871.86 | (2870.5 to 2873.2) ± 6 (Fig. 4b, S9; left and middle) |
| Template-R²-NH₃ | 2954.90 | (2954.5 to 2955.3) ± 6 (Fig. 4b, middle; Fig. S9, left and middle) |
| Template-R¹-NH₃ | 3010.96 | (3009.4 to 3013.7) ± 6 (Fig. 4b, left and middle; Fig. S9, middle) |
| Template-R¹-R²-NH₃ Template-R²-R¹-NH₃ | 3094.00 | 3095.1 ± 6 (Fig. 4b, middle) 3092.4 ± 6 (Fig. S9, middle) |
| Template-R³ | 3096.95 | (3097.2 to 3097.5) ± 6 (Fig. 4b and S9, right; Fig. S8) |
| Template-R²-R³ | 3179.96 | (3179.9 to 3181.4) ± 6 (Fig. 4b and S9, right; Fig. S8, left) |
| Template-R¹-R³ | 3236.02 | (3236.0 to 3237.6) ± 6 (Fig. 4b, S8, S9; right) |
| Template-R¹-R²-R³ Template-R²-R¹-R³ | 3319.06 | 3320.8 ± 6 (Fig. 4b, right) 3319.0 ± 6 (Fig. S9, right) |

*Mismatch Tripeptide Controls*. Reagents **13b** and **14b** were prepared identical to **13** and **14** except that the DNA sequence is scrambled to prevent hybridization. The three-step reaction was run as above with **13b/14/15** or **13/14b/15** and then analyzed by MALDI. In each case, the building block attached to the mismatched reagent was not incorporated in the product **(****FIG. 12****).**

*Three-Step Tripeptide Sequence with Swapped Building Block Order*. Variants of **13** and **14 (13-R²** and **14-R¹)** were prepared that attached R² to **13** and R¹ to **14.** The three-step sequence was performed as above (with aliquots quenched after the 4 °C and 37° C steps) and isolated after streptavidin purification in 38% yield as determined by denaturing PAGE. MALDI-TOF revealed that the order of addition of building blocks is now R² at 4° C and R¹ at 37° C **(****FIG. 13****),** indicating that the DNA sequence (and not the reactants themselves) determines the order of addition of substrates in this system.

*Quantitation of the Tripeptide in the Three-Step Sequence*. While the two major products (R¹-R²-R³ and R¹-R³) of the three-step sequence in **FIG. 4** could not be resolved by denaturing PAGE, the products of the R²-R¹-R³ sequence were separable. Denaturing PAGE of the streptavidin-purified products of a three-step reaction including **13-R², 14-R¹,** and **15** as well as a control reaction with just **13-R²** and **15** that can produce only the dipeptide R²-R³ and monopeptide R³. While the **13-R²/15** reaction runs as a single band, the **13-R²/14-R¹/15** product runs as two bands representing the major tripeptide product and the truncated products lacking R¹. From quantitation of these bands, approximately 55% of the final isolated material was the tripeptide **(****FIG. 14****).** A similar overall purity for the R¹-R²-R³ sequence would be expected but, as stated above, the products of this reaction could not be resolved for a direct quantitation.

## Claims

1. A method of performing in a single reaction mixture multiple sequential nucleic acid-mediated reactions in a pre-selected order, the method comprising:
(a) providing a solution comprising
(i) a first reactive unit and a second reactive unit capable of reacting with one another to form a first reactive intermediate,
(ii) a third reactive unit capable of reacting with the first reactive intermediate to form a second reactive intermediate,
(iii) optionally a fourth reactive unit capable of reacting with the second reactive intermediate, and
(iv) a template oligonucleotide, wherein each of the first, second, third, and optionally fourth reactive units is covalently associated with a corresponding oligonucleotide that is hybridized to the template oligonucleotide and wherein a reaction between the third reactive unit and the first or second reactive units is precluded because the third reactive unit is separated from the first or second reactive units by a duplex formed between the template oligonucleotide and an oligonucleotide covalently associated with a reactive unit, under reaction conditions to bring the first and second reactive units into reactive proximity and induce a pre-selected first reaction between the first and the second reactive units to produce a first reactive intermediate;
(b) adjusting the reaction conditions to dissociate the duplex and bring the third reactive unit and the first reactive intermediate into reactive proximity and induce a pre-selected second reaction between the first reactive intermediate and the third reactive unit to produce a reaction product or a second reactive intermediate; and
(c) optionally, adjusting the reaction conditions to bring the fourth reactive unit and the second reactive intermediate into reactive proximity and induce a pre-selected third reaction between the fourth reactive unit and the second reactive intermediate to produce a reaction product.

2. The method of claim 1 wherein all reactions are mediated by hybridization of the oligonucleotides covalently associated with the reactive units to the template oligonucleotide.

3. The method of claim 1 or claim 2, wherein the reaction condition adjusted in step (b) or (c) is selected from the group consisting of temperature, pH, salt concentration, or a combination of two or more of the foregoing.

4. A method of performing multiple sequential nucleic acid-mediated reactions in a pre-selected order to produce a reaction product, the method comprising:
(a) providing in a single solution (i) a template oligonucleotide defining a first codon, a second codon and a third codon, (ii) a first transfer unit comprising a first reactive unit covalently associated with a first oligonucleotide defining a first anti-codon sequence, (iii) a second transfer unit comprising a second reactive unit covalently associated with a second oligonucleotide defining a second anti-codon sequence, and (iv) a third transfer unit comprising a third reactive unit covalently associated with a third oligonucleotide defining a third anti-codon sequence under conditions so that the oligonucleotides of the first, second, and third transfer units anneal to the template, wherein a duplex created by the template and the first oligonucleotide prevents the first and second reactive units from reacting with the third reactive unit but permits the first and second reactive units to selectively react with one another to produce a reaction intermediate; and
(b) after step (a), adjusting the reaction conditions to disassociate the duplex and permit the third reactive unit to selectively react with the reaction intermediate to produce a reaction product.

5. The method of claim 4, wherein, in step (b), the reaction conditions are adjusted so that step (b) is performed at a higher temperature than step (a).

## Patentansprüche

1. Verfahren zur Ausführung mehrerer aufeinanderfolgender nukleinsäurevermittelter Reaktionen in einer zuvor ausgewählten Reihenfolge in einem einzigen Reaktionsansatz, wobei man in dem Verfahren
(a) eine Lösung bereitstellt, die
(i) eine erste reaktive Einheit und eine zweite reaktive Einheit, die miteinander unter Bildung einer ersten reaktiven Zwischenstufe reagieren können,
(ii) eine dritte reaktive Einheit, die mit der ersten reaktiven Zwischenstufe unter Bildung einer zweiten reaktiven Zwischenstufe reagieren kann,
(iii) gegebenenfalls eine vierte reaktive Einheit, die mit der zweiten reaktiven Zwischenstufe reagieren kann, und
(iv) eine Oligonukleotidmatrize umfasst, wobei die erste, zweite, dritte und gegebenenfalls vierte reaktive Einheit jeweils kovalent mit einem entsprechenden Oligonukleotid, das an die Oligonukleotidmatrize hybridisiert ist, assoziiert ist und wobei eine Reaktion zwischen der dritten reaktiven Einheit und der ersten oder zweiten reaktiven Einheit ausgeschlossen ist, da die dritte reaktive Einheit von der ersten bzw. zweiten reaktiven Einheit durch einen zwischen der Oligonukleotidmatrize und einem kovalent mit einer reaktiven Einheit assoziierten Oligonukleotid gebildeten Duplex getrennt ist, und zwar unter Reaktionsbedingungen, bei denen die erste und die zweite reaktive Einheit in reaktive Nähe zueinander gebracht werden und eine zuvor ausgewählte erste Reaktion zwischen der ersten und der zweiten reaktiven Einheit unter Herstellung einer ersten reaktiven Zwischenstufe induziert wird,
(b) die Reaktionsbedingungen so einstellt, dass der Duplex dissoziiert und die dritte reaktive Einheit sowie die erste reaktive Zwischenstufe in reaktive Nähe zueinander gebracht werden und eine zuvor ausgewählte zweite Reaktion zwischen der ersten reaktiven Zwischenstufe und der dritten reaktiven Einheit unter Herstellung eines Reaktionsprodukts oder einer zweiten reaktiven Zwischenstufe induziert wird, und
(c) gegebenenfalls die Reaktionsbedingungen so einstellt, dass die vierte reaktive Einheit sowie die zweite reaktive Zwischenstufe in reaktive Nähe zueinander gebracht werden und eine zuvor ausgewählte dritte Reaktion zwischen der vierten reaktiven Einheit und der zweiten reaktiven Zwischenstufe unter Herstellung eines Reaktionsprodukts induziert wird.

2. Verfahren nach Anspruch 1, wobei alle Reaktionen durch Hybridisierung der kovalent mit den reaktiven Einheiten assoziierten Oligonukleotide an die Oligonukleotidmatrize vermittelt werden.

3. Verfahren nach Anspruch 1 oder Anspruch 2, wobei die in Schritt (b) oder (c) eingestellte Reaktionsbedingung aus der Gruppe, bestehend aus Temperatur, pH-Wert, Salzkonzentration oder einer Kombination von mindestens zwei dieser Größen, ausgewählt ist.

4. Verfahren zur Ausführung mehrerer aufeinanderfolgender nukleinsäurevermittelter Reaktionen in einer zuvor ausgewählten Reihenfolge zur Herstellung eines Reaktionsprodukts, wobei man in dem Verfahren
(a) in einer einzigen Lösung (i) eine ein erstes Codon, ein zweites Codon und ein drittes Codon definierende Oligonukleotidmatrize, (ii) eine erste Transfereinheit, die eine kovalent mit einem eine erste Anticodonsequenz definierenden ersten Oligonukleotid assoziierte erste reaktive Einheit umfasst, (iii) eine zweite Transfereinheit, die eine kovalent mit einem eine zweite Anticodonsequenz definierenden zweiten Oligonukleotid assoziierte zweite reaktive Einheit umfasst, und (iv) eine dritte Transfereinheit, die eine kovalent mit einem eine dritte Anticodonsequenz definierenden dritten Oligonukleotid assoziierte dritte reaktive Einheit umfasst, unter Bedingungen, so dass die Oligonukleotide der ersten, zweiten und dritten Transfereinheit in einer Annealing-Reaktion an die Matrize binden, wobei ein durch die Matrize und das erste Oligonukleotid erzeugter Duplex die erste und die zweite reaktive Einheit an der Reaktion mit der dritten reaktiven Einheit hindert, jedoch die selektive Reaktion der ersten und der zweiten reaktiven Einheit miteinander unter Herstellung einer Reaktionszwischenstufe gestattet, bereitstellt und
(b) nach Schritt (a) die Reaktionsbedingungen so einstellt, dass der Duplex dissoziiert und der dritten reaktiven Einheit die selektive Reaktion mit der Reaktionszwischenstufe unter Herstellung eines Reaktionsprodukts gestattet wird.

5. Verfahren nach Anspruch 4, wobei in Schritt (b) die Reaktionsbedingungen so eingestellt werden, dass Schritt (b) bei einer höheren Temperatur als Schritt (a) ausgeführt wird.

## Revendications

1. Méthode de réalisation, dans un mélange réactionnel unique, de réactions multiples séquentielles médiées par des acides nucléiques dans un ordre pré-sélectionné, la méthode comprenant :
(a) l'obtention d'une solution comprenant
(i) une première unité réactive et une deuxième unité réactive capables de réagir l'une avec l'autre pour former un premier intermédiaire réactif,
(ii) une troisième unité réactive capable de réagir avec le premier intermédiaire réactif pour former un deuxième intermédiaire réactif,
(iii) éventuellement une quatrième unité réactive capable de réagir avec le deuxième intermédiaire réactif, et
(iv) un oligonucléotide matrice, où chacune des première, deuxième, troisième et éventuellement quatrième unités réactives est associée covalemment à un oligonucléotide correspondant qui est hybridé sur l'oligonucléotide matrice et où une réaction entre la troisième unité réactive et les première ou deuxième unités réactives est écartée car la troisième unité réactive est séparée des première ou deuxième unités réactives par un duplex formé entre l'oligonucléotide matrice et un oligonucléotide associé covalemment à une unité réactive, dans des conditions réactionnelles pour amener les première et deuxième unités réactives dans une proximité réactive et induire une première réaction pré-sélectionnée entre les première et deuxième unités réactives pour produire un premier intermédiaire réactif ;
(b) l'ajustement des conditions réactionnelles pour dissocier le duplex et amener la troisième unité réactive et le premier intermédiaire réactif dans une proximité réactive et induire une deuxième réaction pré-sélectionnée entre le premier intermédiaire réactif et la troisième unité réactive pour produire un produit réactionnel ou un deuxième intermédiaire réactif ; et
(c) éventuellement, l'ajustement des conditions réactionnelles pour amener la quatrième unité réactive et le deuxième intermédiaire réactif dans une proximité réactive et induire une troisième réaction pré-sélectionnée entre la quatrième unité réactive et le deuxième intermédiaire réactif pour produire un produit réactionnel.

2. Méthode selon la revendication 1, **caractérisée en ce que** toutes les réactions sont médiées par hybridation des oligonucléotides associés covalemment aux unités réactives sur l'oligonucléotide matrice.

3. Méthode selon la revendication 1 ou la revendication 2, **caractérisée en ce que** la condition réactionnelle ajustée dans l'étape (b) ou (c) est choisie dans le groupe constitué de la température, du pH, de la concentration de sel, ou d'une combinaison de deux de ce qui précède ou plus.

4. Méthode de réalisation de réactions multiples séquentielles médiées par des acides nucléiques dans un ordre pré-sélectionné pour produire un produit réactionnel, la méthode comprenant :
(a) l'obtention dans une solution unique (i) d'un oligonucléotide matrice définissant un premier codon, un deuxième codon et un troisième codon, (ii) d'une première unité de transfert comprenant une première unité réactive associée covalemment à un premier oligonucléotide définissant une première séquence anti-codon, (iii) d'une deuxième unité de transfert comprenant une deuxième unité réactive associée covalemment à un deuxième oligonucléotide définissant une deuxième séquence anti-codon, et (iv) une troisième unité de transfert comprenant une troisième unité réactive associée covalemment à un troisième oligonucléotide définissant une troisième séquence anti-codon dans des conditions telles que les oligonucléotides des première, deuxième et troisième unités de transfert s'hybrident sur la matrice, un duplex créé par la matrice et le premier oligonucléotide empêchant les première et deuxième unités réactives de réagir avec la troisième unité réactive mais permettant aux première et deuxième unités réactives de réagir sélectivement l'une avec l'autre pour produire un intermédiaire de réaction ; et
(b) après l'étape (a), l'ajustement des conditions réactionnelles pour dissocier le duplex et permettre à la troisième unité réactive de réagir sélectivement avec l'intermédiaire de réaction pour produire un produit réactionnel.

5. Méthode selon la revendication 4, **caractérisée en ce que,** dans l'étape (b), les conditions réactionnelles sont ajustées pour que l'étape (b) soit effectuée à une température plus élevée que l'étape (a).
